# EUROPÄISCHE PATENTSCHRIFT

(11) **EP 4 633 550 B1**
(45) Veröffentlichungstag und Bekanntmachung des Hinweises auf die Patenterteilung: **22.07.2026**
(21) Anmeldenummer: 23821624.6
(22) Anmeldetag: 11.12.2023
(51) Int. Cl.: A61F 5/01

(54) **REVERSIBEL SCHLIESSBARE EXTREMITÄTENORTHESE ODER -BANDAGE ODER SPORTHILFSMITTEL**
REVERSIBLY CLOSABLE LIMB ORTHOSIS OR LIMB BANDAGE OR SPORTS AID DEVICE
ORTHÈSE DE MEMBRE OU BANDAGE DE MEMBRE OU DISPOSITIF D'AIDE AU SPORT POUVANT ÊTRE FERMÉ DE MANIÈRE RÉVERSIBLE

(30) Priorität: 13.12.2022 DE 102022213570
(43) Veröffentlichungstag der Anmeldung: 22.10.2025
(73) Patentinhaber: Bauerfeind AG, 07937 Zeulenroda-Triebes (DE)
(72) Erfinder: STIER, Gerald, 07937 Zeulenroda-Triebes (DE); HEBENSTREIT, Sandro, 07937 Zeulenroda-Triebes (DE); BAUERFEIND-JOHNSON, Beatrix, 07937 Zeulenroda-Triebes (DE)
(74) Vertreter: Gleiss Große Schrell und Partner mbB
(86) Internationale Anmeldenummer: PCT/EP2023/085097
(87) Internationale Veröffentlichungsnummer: WO 2024/126360

(56) Entgegenhaltungen:
- US-A1- 2016 095 734
- US-B2- 8 277 401

## Beschreibung

Die vorliegende Erfindung betrifft eine Extremitätenorthese oder -bandage oder ein Sporthilfsmittel, umfassend ein Spannsystem aus einem ersten Spannteilsystem und einem zweiten Spannteilsystem wobei das erste Spannteilsystem und das zweite Spannteilsystem jeweils reversibel schließbar sind.

Orthesen und Bandagen für Extremitäten, auch Extremitätenorthese oder -bandage genannt, insbesondere Knieorthesen zur passiven und aktiven Stabilisierung des Kniegelenks, aber auch Ellenbogenorthesen sind aus dem Stand der Technik bekannt. Solche Knieorthesen weisen häufig ein Trägerelement in Form einer Bandage auf, auf das Stabilisierungselemente wie Gurte, Stäbe oder Gelenkschienen montiert sind, wie es beispielsweise in der DE 200 05 366 U1 gezeigt ist.

Solche Knieorthesen werden beispielsweise bei einer Kniearthrose oder nach Verletzung des Innenmeniskus eingesetzt, um den betroffenen Bereich zu entlasten und zu stabilisieren. Knieorthesen weisen dabei häufig Gurte auf, mit denen die Knieorthese eingestellt werden, also an die Beinform und/oder auf den Wirkungsbereich angepasst werden kann. Spezifisch geführte Spannsysteme sind beispielsweise aus der EP 2 612 626 A2, der DE 40 136 93 A1, der DE 10 2017 220 968 A1, der WO 1996 / 16615 A1 und der DE 198 44 545 A1 bekannt. Die US 8 277 401 B2 offenbart eine Orthese mit einem Spannsystem aus zwei Spannteilsystemen.

Diese Spannsysteme bedingen, dass der Träger, insbesondere der Patient beim Anziehen der Orthese oder Bandage in das Spannsystem mit der Extremität, insbesondere mit dem Bein, hineinsteigen muss und beim Ausziehen heraussteigen muss, was kompliziert und insbesondere für Patienten unangenehm und gefährlich sein kann.

Das der vorliegenden Erfindung zugrunde liegende Problem ist die Bereitstellung einer Extremitätenorthese oder -bandage, insbesondere Knieorthese oder -bandage, die die Probleme aus dem Stand der Technik überwindet und insbesondere ein einfacheres und sichereres An- und ausziehen der Orthese oder Bandage ermöglicht.

Die vorliegende Erfindung löst das ihr zugrunde liegende technische Problem durch eine Extremitätenorthese oder -bandage oder ein Sporthilfsmittel nach Anspruch 1.

Alle folgenden Ausführungsformen für eine erfindungsgemäße Extremitätenorthese oder Extremitätenbandage sind auch für erfindungsgemäße Sporthilfsmittel offenbart.

Es zeigte sich, dass es in vorteilhafter Weise möglich ist ein voll funktionsfähiges Spannsystem bereitzustellen, das reversibel verschließbar und öffenbar ist. Dadurch kann der Träger, insbesondere Patient in vorteilhafter Weise beim Anziehen der Orthese oder Bandage das Spannsystem um die Extremität, insbesondere um das Bein, legen und schließen, so dass nicht mit dem Bein in das Spannsystem eingestiegen werden muss oder der Arm eingeführt werden muss. Das Gleiche gilt beim Entfernen der Orthese oder Bandage, bei dem das Spannsystem wieder geöffnet werden kann und so das Spannsystem abgezogen werden kann, ohne dass der Träger, insbesondere Patient die Extremität aus dem Spannsystem herausziehen muss.

In einer bevorzugten Ausführungsform sind bei der angelegten Extremitätenorthese oder - bandage das erste Spannteilsystem und/oder das zweite Spannteilsystem des Spannsystems so um die Extremität, insbesondere ein Bein, geführt, dass das erste Spannteilsystem und/oder das zweite Spannteilsystem des Spannsystems jeweils die Extremität umschließt. Erfindungsgemäß ist insbesondere vorgesehen, dass das erste Spannteilsystem dem oberen Teil der Extremität zugeordnet ist, also dem Oberschenkel oder Oberarm, und das zweite Spannteilsystem dem unteren Teil der Extremität zugeordnet ist, also dem Unterschenkel oder Unterarm, so dass Knie oder Ellenbogen im angezogenen Zustand zwischen den beiden Spannteilsystem positioniert sind.

In einer bevorzugten Ausführungsform werden das erste Spannteilsystem und das zweite Spannteilsystem aus einem durchgehenden Spannelement gebildet. Es ist also in vorteilhafter Weise möglich, die erfindungsgemäße Extremitätenorthese oder -bandage mit nur einem einzigen Spannelement zu bilden.

Alternativ kann natürlich aber auch vorgesehen sein, dass das erste Spannteilsystem aus einem ersten Spannelement gebildet wird und das zweite Spannteilsystem aus einem zweiten Spannelement gebildet wird.

In einer bevorzugten Ausführungsform ist das mindestens eine Spannelement ein Gurt, ein Flachband, ein Seil, ein Draht oder ein Bowdenzug. In einer bevorzugten Ausführungsform ist das mindestens eine Spannelement ein Seil, ein Draht oder ein Bowdenzug.

Das Spannelement kann flexibel oder auch unflexibel oder teilweise flexibel ausgestaltet sein. In einer bevorzugten Ausführungsform ist das mindestens eine Spannelement unelastisch.

In einer bevorzugten Ausführungsform umfasst das mindestens eine Spannelement ein Seil oder einen Draht, wobei das Seil oder der Draht zumindest bereichsweise in einer Ummantelung verläuft, wobei die Ummantelung an der Extremitätenorthese oder -bandage verschiebbar gelagert ist.

Der Bowdenzug und somit auch das Seil können auf einer Polsterung zumindest abschnittsweise befestigt sein. Die Polsterung, beispielsweise auch als Gurt oder Riemen ausgebildet, dient dem in vorteilhafter Weise höheren Tragekomfort für den Patienten und soll ein "einschneiden" des Seils in die Extremität verhindern. Die Polsterung kann elastisch wie auch unelastisch sein. Es sind auch Polsterungen möglich, welche nur teilweise elastische Bereiche aufweisen.

Das mindestens eine Spannelement kann auch über drehbar gelagerte Befestigungselemente mit der Polsterung verbunden werden. In der bevorzugten Ausgestaltung sind die Polsterung sowie die gesamte Seilführung leicht elastisch. In einer bevorzugten Ausführungsform weist die Extremitätenorthese oder -bandage drehbar gelagerte Umlenkungsführungen auf, in denen das mindestens eine Spannelement umgelenkt wird.

In einer bevorzugten Ausführungsform sind das erste Spannteilsystem durch eine erste Kopplungsvorrichtung und das zweite Spannteilsystem durch eine zweite Kopplungsvorrichtung reversibel schließbar.

Erfindungsgemäß sind das erste Spannteilsystem und das zweite Spannteilsystem jeweils durch eine Kopplungsvorrichtung reversibel schließbar, wobei jede Kopplungsvorrichtung ein erstes Kopplungselement und ein zweites Kopplungselement umfasst, wobei das erste Kopplungselement mit dem zweiten Kopplungselement über ein Rastsystem reversibel miteinander verbindbar sind, wobei das erste Kopplungselement eine erste Führung für ein Spannelement aufweist und das zweite Kopplungselement eine zweite Führung für ein Spannelement aufweist, wobei das Spannelement in der ersten Führung verläuft und das Spannelement in der zweiten Führung verläuft und wobei im verbundenen Zustand der beiden Kopplungselemente sich die erste Führung und die zweite Führung mindestens einmal, insbesondere zweimal kreuzen.

Durch das mindestens einmalige, insbesondere zweimalige Kreuzen der Führungen kreuzen sich auch die darin befindlichen Spannelemente. Werden also die beiden Kopplungselemente miteinander verbunden, beispielsweise um die Orthese zu schließen, so überkreuzen sich die beiden in ihnen geführten Spannelemente in zwei unterschiedlichen Ebenen. Das Überkreuzen der Spannelemente hat den Vorteil, dass die Kopplungselemente auf relativ kleinem Raum verwirklicht werden können und die Reibung durch diesen Aufbau minimiert wird. Dies führt außerdem zu einer besseren Kraftverteilung innerhalb des gesamten Spannsystems.

Eine bevorzugte zweimalige Kreuzung der Führungen kommt zum Beispiel zustande, wenn die beiden Führungen gegenläufig gekrümmt sind und überlappen. Eine einmalige Kreuzung der Führungen kommt zum Beispiel zustande, wenn die beiden Führungen gegenläufig gekrümmt sind und nur an ihren Spitzen, also den Scheitelpunkten der Krümmung überlappen.

Bevorzugt ist, dass die erste Führung eine Krümmung aufweist, insbesondere hufeisenförmig ist und dass die zweite Führung eine Krümmung aufweist, insbesondere hufeisenförmig ist. Bevorzugt überlappen sich die beiden hufeisenförmigen Führungen im verbundenen Zustand der beiden Kopplungselemente. Bevorzugt zeigen die beiden Enden der ersten hufeisenförmigen Führung von der zweiten hufeisenförmigen Führung weg und die beiden Enden der zweiten hufeisenförmigen Führung zeigen von der ersten hufeisenförmigen Führung weg.

Die Führungen können beispielsweise als Tunnel oder Kanal, auch als durchbrochener Tunnel oder Kanal ausgestaltet sein. Durch die Führungen können sich die Spannelemente berührungsfrei kreuzen.

In einer bevorzugten Ausführungsform ist die erste Führung im Randbereich des ersten Kopplungselements positioniert und die zweite Führung ist im Randbereich des zweiten Kopplungselements positioniert.

In einer bevorzugten Ausführungsform weist das erste Kopplungselement eine Vorderseite und eine Rückseite auf und weist eine Hufeisenform mit einer konkaven Innenkante auf, wobei das zweite Kopplungselement eine Vorderseite und eine Rückseite aufweist und eine Hufeisenform mit einer konkaven Innenkante aufweist, wobei das erste Kopplungselement im Bereich der Spitze der Hufeisenform auf der Vorderseite ein erstes Rastelement aufweist und das zweite Kopplungselement im Bereich der Spitze der Hufeisenform auf der Rückseite ein zweites Rastelement aufweist und wobei das zweite hufeisenförmige Kopplungselement ein die konkave Innenkante des zweiten Kopplungselements überspannendes erstes Stegelement aufweist, wobei im verbundenen Zustand der beiden Kopplungselemente das erste Rastelement des ersten Kopplungselements in das erste Stegelement des zweiten Kopplungselements eingreift und das zweite Rastelement des zweiten Kopplungselements in die konkave Innenkante des ersten Kopplungselements eingreift.

Bevorzugt ist, dass das erste Kopplungselement und das zweite Kopplungselement im verbundenen Zustand der beiden Kopplungselemente gegeneinander verschiebbar beziehungsweise verdrehbar sind. Dies hat den Vorteil, dass auch die Spannelemente gegeneinander verschiebbar sind.

Bevorzugt ist, dass das zweite Kopplungselement ein zweites die konkave Innenkante des zweiten Kopplungselements überspannendes Stegelement aufweist, das im verbundenen Zustand der beiden Kopplungselemente das erste Stegelement gegen das erste Rastelement drückt. Bevorzugt weist das zweite Stegelement in seinem Mittelbereich eine schräge Abflachung auf, die insbesondere beim Biegen unter den ersten Steg gleiten kann und diesen hochhebt oder auf andere Weise von dem Rastelement löst.

Bevorzugt ist, dass das erste Kopplungselement und das zweite Kopplungselement aus Kunststoff gefertigt sind.

Bevorzugt ist, dass die erste Führung und/oder die zweite Führung als Griffelement ausgebildet sind.

Bevorzugt ist auch eine beschriebene Kopplungsvorrichtung, wobei das erste Kopplungselement mit dem zweiten Kopplungselement über das Rastsystem reversibel miteinander verbunden ist und die Kopplungsvorrichtung dadurch geöffnet wird, dass das zweite hufeisenförmige Kopplungselement an den Armen des hufeisenförmigen Kopplungselement zusammengedrückt wird, wodurch bevorzugt das zweite die konkave Innenkante des zweiten Kopplungselements überspannende Stegelement in Richtung Spitze des zweiten hufeisenförmigen Kopplungselements und/oder in Richtung des ersten Kopplungselements geschoben wird und wodurch das zweite die konkave Innenkante des zweiten Kopplungselements überspannendes Stegelement bevorzugt das erste Stegelement aus dem ersten Rastelement heraushebt, so dass bevorzugt die beiden Kopplungselemente gegeneinander geschoben werden und dadurch voneinander getrennt werden können.

In einer bevorzugten Ausführungsform weist die Extremitätenorthese oder -bandage ein Verbindungselement auf, an dem das mindestens eine Spannelement, insbesondere das einzige Spannelement oder das erste Spannelement und/oder das zweite Spannelement befestigt sind.

In einer bevorzugten Ausführungsform ist das mindestens eine Spannelement mit beiden Enden am Verbindungselement befestigt. In einer bevorzugten Ausführungsform ist das eine, also das einzige Spannelement mit beiden Enden am Verbindungselement befestigt. Bei mehreren Spannelementen kann auch vorgesehen sein, dass diese mit jeweils nur einem Ende am Verbindungselement befestigt sind.

In einer bevorzugten Ausführungsform weist das Verbindungselement ein Spannungsregelelement zum Spannen des mindestens einen Spannelements auf. In einer bevorzugten Ausführungsform ist das Verbindungselement ein Spannungsregelelement zum Spannen des mindestens einen Spannelements.

In vorteilhafter Weise ist also vorgesehen, dass die beiden Spannteilsysteme zusammen oder getrennt gespannt werden können, sodass sie zum einen einen festen Sitz der Orthese oder Bandage für Extremitäten an der Extremität, insbesondere der Knieorthese oder -bandage an dem Bein ermöglichen und zum anderen eine gute Positionierung und einen guten Sitz einer bevorzugten Gelenkschiene ermöglichen.

In einer bevorzugten Ausführungsform ist das Spannungsregelelement als Drehknopf ausgebildet, mit dem das Spannelement gespannt werden kann oder die zwei Spannelemente gleichzeitig gespannt werden können. Bevorzugt werden die Spannelemente also durch Drehen eines Knopfes gespannt, beispielsweise indem durch den Drehknopf Seile aufgewickelt werden, die die Spannelemente bilden. Es ist also bevorzugt vorgesehen, dass die beiden Spannteilsysteme gleichzeitig durch ein Spannungsregelelement gespannt werden, sodass die beiden Spannteilsysteme die entsprechend gleiche Spannkraft haben und somit die beiden Spannteilsysteme zu einem einzigen Spannsystem zusammengeführt sind und nur ein Arbeitsschritt zum Spannen beider Spannteilsysteme notwendig ist.

Natürlich ist das Spannungsregelelement so ausgebildet, dass die Spannteilsysteme auch wieder entspannt werden können. Beispielsweise durch eine Rasterung im Drehknopf können bevorzugt verschiedene Spannungsgrade eingestellt werden, so dass in vorteilhafter Weise eine spezifische Krafteinstellung möglich ist.

In einer bevorzugten Ausführungsform ist das mindestens eine Spannelement mit beiden Enden am Spannungsregelelement befestigt. Es kann aber auch mit nur einem Ende am Spannungsregelelement befestigt sein. Das zweite Ende kann dann an einem beliebigen Punkt der Orthese befestigt sein.

Es kann auch ein erstes Spannelement für das erste Spannteilsystem und ein zweites Spannelement für das zweite Spannteilsystem vorgesehen sein, wobei jedes der zwei Spannelemente mit einem Ende an dem Spannungsregelelement befestigt ist und mit dem zweiten Ende an einem beliebigen Punkt der Orthese oder Bandage, beispielsweise an einer Gelenkschiene.

In einer bevorzugten und vorteilhaften Ausführungsform ist das Spannsystem so geführt, dass sich das erste Spannteilsystem in einem ersten Kreuzungsbereich kreuzt und dass sich das zweite Spannteilsystem in einem zweiten Kreuzungsbereich kreuzt. Bevorzugt wird der erste Kreuzungsbereich durch die erste Kopplungsvorrichtung und der zweite Kreuzungsbereich durch die zweite Kopplungsvorrichtung gebildet.

Bevorzugt liegt der erste und/oder der zweite Kreuzungsbereich im getragenen Zustand auf der Extremitäteninnenseite. Bevorzugt liegt die erste und/oder die zweite Kopplungsvorrichtung im getragenen Zustand auf der Extremitäteninnenseite. Alternativ können die Kreuzungsbereiche und oder die Kopplungsvorrichtungen auf der Extremitätenaußenseite liegen oder hinten liegen oder vorne liegen.

Bevorzugt ist also bei der angelegten Extremitätenorthese oder -bandage, dass das erste Spannteilsystem und/oder das zweite Spannteilsystem des Spannsystems so um die Extremität, insbesondere ein Bein, geführt sind, dass das erste Spannteilsystem und/oder das zweite Spannteilsystem des Spannsystems die Extremität umschlingt und sich dabei einmal kreuzt.

Die vorliegende Erfindung betrifft auch eine Extremitätenorthese oder -bandage, umfassend ein Spannsystem aus einem ersten Spannteilsystem und einem zweiten Spannteilsystem, wobei die beiden Spannteilsysteme bevorzugt aus einem einzigen Spannelement gebildet werden und wobei das Spannsystem so geführt ist, dass sich das erste Spannteilsystem in einem ersten Kreuzungsbereich kreuzt und dass sich das zweite Spannteilsystem in einem zweiten Kreuzungsbereich kreuzt, wobei der erste und/oder der zweite Kreuzungsbereich im getragenen Zustand auf der Extremitäteninnenseite liegt/liegen und wobei bevorzugt beide Enden des Spannelements mit einem Spannungsregelelement verbunden sind. Diese Extremitätenorthese oder -bandage kann reversibel schließbar sein oder nicht reversibel schließbar sein.

Eine bevorzugte Führung des Spannelements bei einer erfindungsgemäßen Extremitätenorthese mit nur einem Spannelement ist im getragenen Zustand folgende:
Das Spannelement startet mit dem ersten Ende in etwa auf Höhe des Knies oder des Ellenbogens am Spannungsregelelement und verläuft nach oben über den Oberschenkel oder den Oberarm. Danach verläuft das Spannelement bis zu einer Seite der Extremität, insbesondere bis zur Innenseite der Extremität und wird dort durch das erste Kopplungselement des ersten Kopplungssystems des ersten Spannteilsystems nach oben umgelenkt, worauf das Spannelement erneut über den Oberschenkel oder Oberarm verläuft. Dann gleitet das Spannelement über den vorderen Bereich des Oberschenkels oder Oberarms zur anderen Seite der Extremität, insbesondere der Außenseite der Extremität und verläuft über der Hinterseite des Oberschenkels oder Oberarms zur ersten Seite, insbesondere Innenseite des Oberschenkels oder Oberarms. Dort wird das Spannelement im zweiten Kopplungselement des ersten Kopplungssystems nach unten umgelenkt und in Richtung hinterer Oberschenkel oder Oberarm weitergeführt. Durch das geschlossene erste Kopplungssystem kreuzt sich so das Spannelement zweimalig. Das Spannelement verläuft leicht schräg nach unten am hinteren Oberschenkel oder Oberarm zur zweiten Seite, insbesondere Außenseite der Extremität und wird dort senkrecht nach unten unter das Gelenk, also das Knie oder den Ellenbogen, zum Unterschenkel oder Unterarm und somit zum zweiten Spannteilsystem geführt. Dort verläuft das Spannelement über der Hinterseite des Unterschenkels oder Unterarms zur ersten Seite, insbesondere Innenseite des Unterschenkels oder Unterarms. Dort wird das Spannelement im zweiten Kopplungselement des zweiten Kopplungssystems des zweiten Spannteilsystems nach unten umgelenkt und in Richtung hinterer Unterschenkel oder Unterarm weitergeführt. Das Spannelement verläuft leicht schräg am hinteren Unterschenkel oder Unterarm zur zweiten Seite, insbesondere Außenseite der Extremität und wird dort in Richtung vorderer Unterschenkel oder Unterarm und von da weiter zur ersten Seite, insbesondere Innenseite des Unterschenkels oder Unterarms geführt. Dort wird das Spannelement durch das erste Kopplungselement des zweiten Kopplungssystems des zweiten Spannteilsystems nach oben umgelenkt, worauf das Spannelement erneut über die Vorderseite des Unterschenkels oder Unterarms verläuft und mit dem zweiten Ende wieder am Spannungsregelelement endet. Mit dem Spannungsregelelement können somit beide Enden des Spannelements gespannt werden, beispielsweise durch Aufrollen gespannt werden.

Bevorzugt liegt das Spannungsregelelement auf der Außenseite der Extremität, insbesondere wenn die Kopplungssysteme auf der Innenseite liegen. Alternativ liegt das Spannungsregelelement auf der Innenseite der Extremität, insbesondere wenn die Kopplungssysteme auf der Außenseite liegen.

In einer bevorzugten Ausführungsform weist eine erfindungsgemäße Extremitätenorthese oder - bandage mindestens eine Gelenkschiene, bevorzugt zwei Gelenkschienen auf. Bevorzugt sind die zwei Gelenkschienen im getragenen Zustand seitlich positioniert, also eine an der Außenseite der Extremität und eine an der Innenseite der Extremität. Bevorzugt weist die Extremitätenorthese oder -bandage eine Gelenkschiene auf, die im getragenen Zustand auf der Außenseite der Extremität positioniert ist. Alternativ weist die Extremitätenorthese oder - bandage eine Gelenkschiene auf, die im getragenen Zustand auf der Innenseite der Extremität positioniert ist. Bevorzugt ist bei der Verwendung von zwei Gelenkschienen, wobei eine der beiden Gelenkschienen, insbesondere die auf der Außenseite der Extremität positionierte Gelenkschiene, mit dem Spannregelelement verbunden ist. Bevorzugt ist eine zweite Gelenkschiene, insbesondere die Gelenkschiene auf der Innenseite der Extremität mit einem der beiden Kopplungselemente des ersten Spannteilelements und einem der beiden Kopplungselemente des zweiten Spannteilelements verbunden.

Gelenkschienen sind meist so ausgeführt, dass nicht nur in der sagittalen Ebene die Kniebewegung, sondern auch in der Frontalebene eine Einstellung stattfinden kann. Dabei handelt es sich in der Frontalebene um einstellbare Scharniergelenke, die sich so nahe wie möglich am Krümmungsscheitel des Kniegelenks oder Ellenbogengelenks befinden, um so im Sinne einer Entlastung des inneren Kompartiments des Femortibialgelenks oder Ellenbogengelenks zu wirken. Die im Stand der Technik gezeigten zirkulär angeordneten Gurte wirken als Gegenlager oberhalb und unterhalb des Kniegelenks auf der gegenüberliegenden Seite der Gelenkschiene. Solche Gelenkschienen sind beispielsweise aus der WO 2007/145504 A1 oder der WO 2003/103547 A1 bekannt.

In einer bevorzugten Ausführungsform weist die mindestens eine Gelenkschiene einen ersten Schienenabschnitt, einen zweiten Schienenabschnitt und einen dritten Schienenabschnitt auf, wobei der erste Schienenabschnitt und der zweite Schienenabschnitt jeweils durch ein Gelenk mit dem dritten Schienenabschnitt verbunden sind.

In einer bevorzugten Ausführungsform sind die Schienenabschnitte elastisch ausgestaltet. In einer bevorzugten Ausführungsform wird der dritte Schienenabschnitt aus einem Federelement, insbesondere einer Blattfeder, gebildet. Auch die beiden anderen Schienenabschnitte können aus Blattfedern gebildet sein.

In einer bevorzugten Ausführungsform sind die Gelenke monozentrische Gelenke. Die zwei monozentrischen Gelenke oberhalb und unterhalb des dritten, mittleren Schienenabschnitts einer Gelenkschiene ergeben zusammen eine Duo-Zentrik, die in vorteilhafter Weise dazu führt, dass eine Beugung des Knies weniger Stress im Kniegelenk erzeugt. Während der Beugung kann das Drehzentrum mehrfach von einem zum anderen Drehpunkt wechseln. In Kombination mit elastischen Schienen, insbesondere der Blattfeder, sorgt die Duo-Zentrik dafür, dass die Orthese oder Bandage sich in der Beugung nicht seitlich gegen die Extremitätenachse, insbesondere Beinachse verschiebt.

Die Ausbildung des dritten Schienenabschnitts aus einem Federelement, insbesondere aus einer Blattfeder, beispielsweise aus Federstahl, führt dazu, dass der erste Schienenabschnitt und der zweite Schienenabschnitt nicht nur in einer Achse rotieren, sondern auch in einer um 90° gedrehten Achse beweglich sind, und zwar bevorzugt in dem Bereich, in dem durch das Kopplungselement der mittlere Krafteinleitungspunkt des durch das Spannsystem hergestellte Dreipunktsystems flexibel ist. Darüber hinaus führen die beiden Gelenke, die den ersten Schienenabschnitt beziehungsweise den zweiten Schienenabschnitt mit dem dritten Schienenabschnitt verbinden, dazu, dass sich die Gelenkschiene als solche viel besser an die Konturen des Körpers anpassen kann. Auch führt die Federung des dritten Schienenabschnitts vorteilhafterweise dazu, dass die Orthese oder Bandage für eine Extremität, insbesondere Knieorthese bei extremen Bewegungen etwas nachgeben kann und dabei den Benutzer vor einer übermäßig falschen Beinbewegung warnt.

In einer bevorzugten Ausführungsform ist das Verbindungselement am dritten Schienenabschnitt der mindestens einen Gelenkschiene, insbesondere einer der zwei Gelenkschienen befestigt.

Bei der Orthese oder Bandage für Extremitäten, insbesondere Knieorthese oder Kniebandage kann zusätzlich ein Trägerelement vorhanden sein, beispielsweise ein Bandagengrundelement, insbesondere ein Gestrick oder ein Gewirk, es kann aber durch den Verlauf des erfindungsgemäßen Spannsystems auch auf das Trägerelement verzichtet werden.

Die Extremitätenorthese oder -bandage kann zusätzlich einen Bandagengrundkörper aufweisen. Geeignete Bandagengrundkörper, insbesondere Schlauchbandagengrundkörper, sind dem Fachmann bekannt, Der Bandagengrundkörper ist bevorzugt nicht dauerhaft mit der restlichen Extremitätenorthese oder -bandage verbunden, sondern gar nicht oder reversibel mit der restlichen Extremitätenorthese oder -bandage verbunden. Dies ermöglicht, dass der Bandagengrundkörper in herkömmlicher Weise über die Extremität gezogen werden kann und danach die restliche Extremitätenorthese oder -bandage in der beschriebenen vorteilhaften Weise angelegt werden kann. In einer alternativen Ausführungsform kann auch der Bandagengrundkörper fest und dauerhaft mit der restlichen Extremitätenorthese oder -bandage verbunden sein, beispielsweise, wenn sich auch der Bandagengrundkörper öffnen lässt, zum Beispiel durch einen Reisverschluss, durch einen Klettverschluss oder durch Knöpfe, und so auch der Bandagengrundkörper beim Anziehen um die Extremität gelegt und dann geschlossen werden kann und der Träger nicht die Extremität durch den Bandagengrundkörper hindurchstecken muss.

Natürlich kann aber auch vorgesehen sein, keinen Bandagengrundkörper zu verwenden, so dass die Extremitätenorthese oder Bandage direkt auf der Haut oder auf einem Kleidungsstück getragen wird. Somit kann die Orthese oder Bandage dem Therapieverlauf des Patienten angepasst werden.

Ob es sich bei einem entsprechenden Produkt um eine Orthese oder Bandage handelt, hängt davon ab ob das Produkt ein bandagenartiges Gestrick oder Gewirk aufweist und/oder wie stark das Produkt aufgerüstet ist.

In einer bevorzugten Ausführungsform ist die Extremitätenorthese oder -bandage eine Knieorthese oder -bandage.

In einer bevorzugten Ausführungsform ist die Extremitätenorthese eine Knieorthese. Insbesondere bei einer Knieorthese ist das einfachere Anlegen und Ablegen der Orthese vorteilhaft. Die Vorteile sind aber auch bei einer Ellenbogenorthese vorhanden, so dass es sich bei der Extremitätenorthese auch um eine Ellenbogenorthese handeln kann.

In einer bevorzugten Ausführungsform ist die Extremitätenbandage eine Kniebandage. Insbesondere bei einer Kniebandage ist das einfachere Anlegen und Ablegen der Bandage vorteilhaft. Die Vorteile sind aber auch bei einer Ellenbogenbandage vorhanden, so dass es sich bei der Extremitätenbandage auch um eine Ellenbogenbandage handeln kann.

Die erfindungsgemäße Extremitätenorthese oder -bandage kann auch als Sporthilfsmittel verwendet werden. In einer alternativen Ausführungsform ist die erfindungsgemäße Extremitätenorthese oder -bandage ein Sporthilfsmittel.

Wenn in der vorliegenden Beschreibung auf eine Position eines Elementes der Orthese oder der Bandage zu einem Körperteil verweisen wird, so bezieht sich dies auf die Orthese oder Bandage im getragenen Zustand, also wenn die Orthese oder Bandage an die Extremität angelegt ist.

Die Erfindung wird anhand eines Beispiels und der Figuren näher erläutert, wobei diese nicht einschränkend zu verstehen sind.

Es zeigen
- Figur 1: eine erfindungsgemäße Extremitätenorthese als Knieorthese,
- Figur 2 bis Figur 7: verschiedene Perspektiven der Knieorthese aus Figur 1,
- Figuren 8 bis 11: Detailansichten von der Knieorthese aus Figur 1,
- Figur 12: eine erfindungsgemäße Extremitätenorthese als Ellenbogenorthese,
- Figur 13: die Ellenbogenorthese aus Figur 12,
- Figur 14: eine erfindungsgemäße Extremitätenorthese als Ellenbogenorthese mit Bandage,
- Figur 15: die Ellenbogenorthese aus Figur 14,
- Figur 16: eine bevorzugte Kopplungsvorrichtung für eine erfindungsgemäße Orthese in geschlossenem Zustand von vorne,
- Figur 17: die Kopplungsvorrichtung aus Figur 16 in geschlossenem Zustand von hinten,
- Figur 18: die Kopplungsvorrichtung aus Figur 16 in offenem Zustand von vorne,
- Figur 19: die Kopplungsvorrichtung aus Figur 16 in offenem Zustand von hinten,
- Figur 20: die Kopplungsvorrichtung aus Figur 16 mit Angaben zur Benutzung,
- Figur 21: Detailansichten der Kopplungsvorrichtung aus Figur 16.

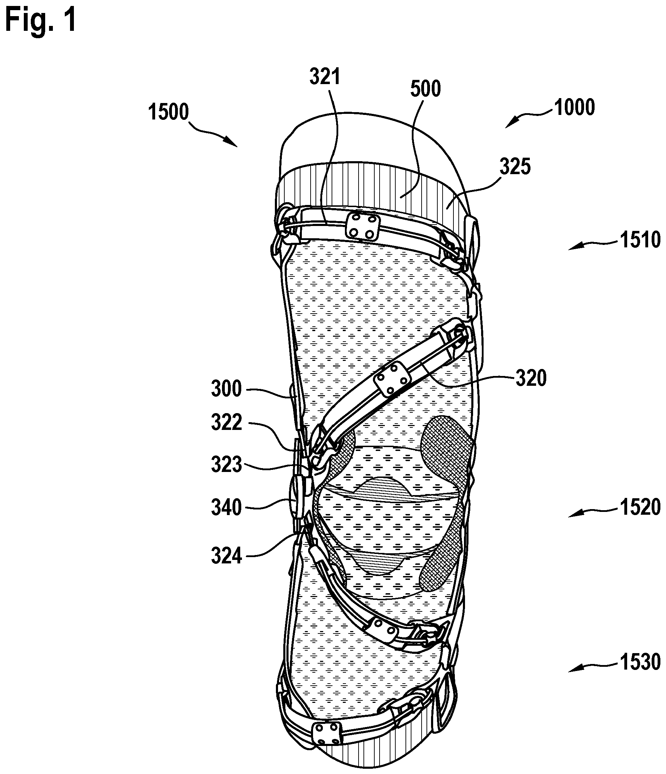

Figur 1 zeigt eine erfindungsgemäße Extremitätenorthese als Knieorthese (1000) an einem Bein (1500) mit Oberschenkel (1510), Kniebereich (1520) und Unterschenkel (1530). Der Knieorthese (1000) ist eine Bandage (500) zugeordnet. Die Knieorthese umfasst ein Spannsystem (300) aus einem Bowdenzug (320) mit einem durch eine Ummantelung (321) größtenteils verdeckten Seil (323). Der Bowdenzug (320) verläuft auf einer Polsterung (325). Das erste Seilende (322) und das zweite Seilende (324) sind mit einem Drehknopf (340) zum Spannen verbunden.

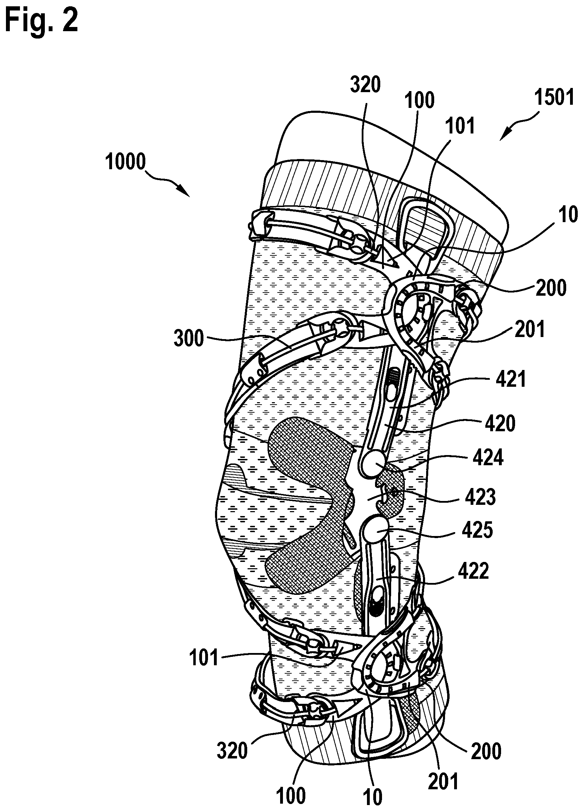

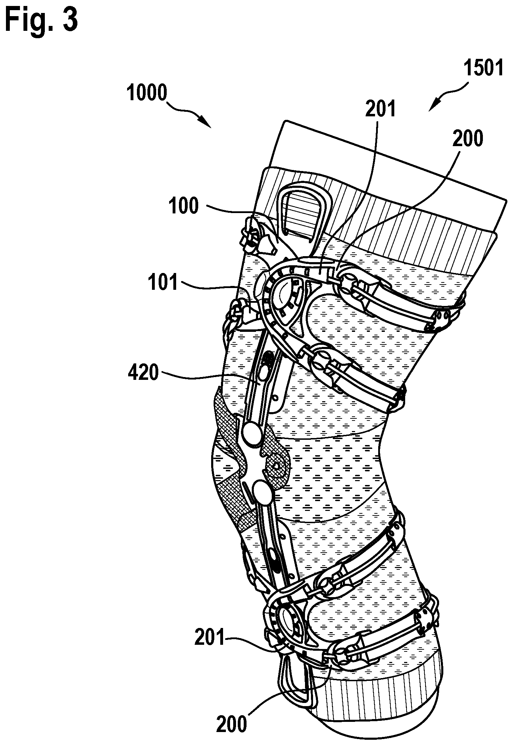

In Figur 2 und Figur 3 ist die Knieorthese (1000) an der Innenseite (1501) des Beins zu sehen. Hier verläuft eine zweite Gelenkschiene (420) mit einem ersten Schienenabschnitt (421), der über ein erstes Gelenk (424) mit einem dritten Schienenabschnitt (423) verbunden ist und einem zweiten Schienenabschnitt ((422), der auch über ein Gelenk (425) mit dem dritten Schienenabschnitt (423) verbunden ist. Im Bereich der zweiten Gelenkschiene (420) befinden sich zwei Kopplungsvorrichtungen (10) aus jeweils einem ersten Kopplungselement (100) und einem zweiten Kopplungselement (200). Die zwei Kopplungsvorrichtungen (10) erfüllen gleichzeitig zwei Funktionen, nämlich zum einen die Möglichkeit die Knieorthese (1000) reversibel öffnen und schließen zu können und zum anderen den Bowdenzug (320) jeweils über eine erste Führung (10) und eine zweite Führung (201) umzulenken.

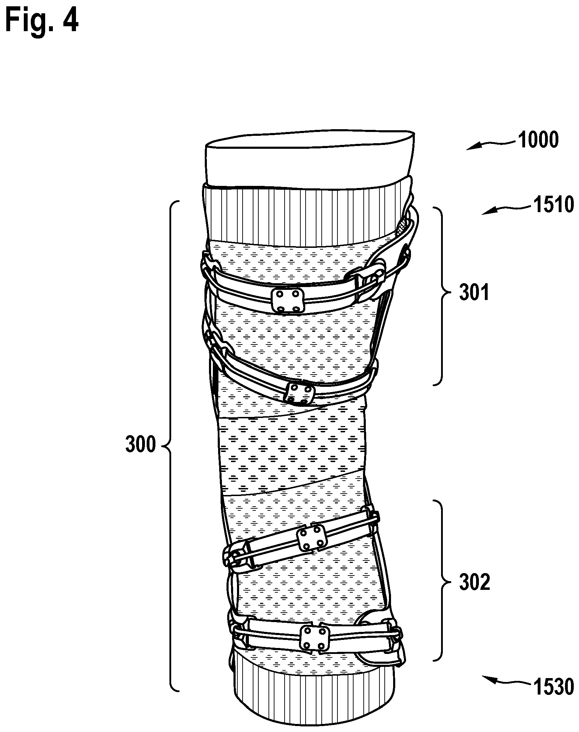

Figur 4 zeigt die Knieorthese (1000) an der Rückseite des Beins. Hier ist zu sehen wie sich das Spannsystem (300) in ein erstes Spannteilsystem (301) am Oberschenkel (1510) und ein zweites Spannteilsystem (302) am Unterschenkel (1530) aufgeteilt ist, die beide gespannt werden.

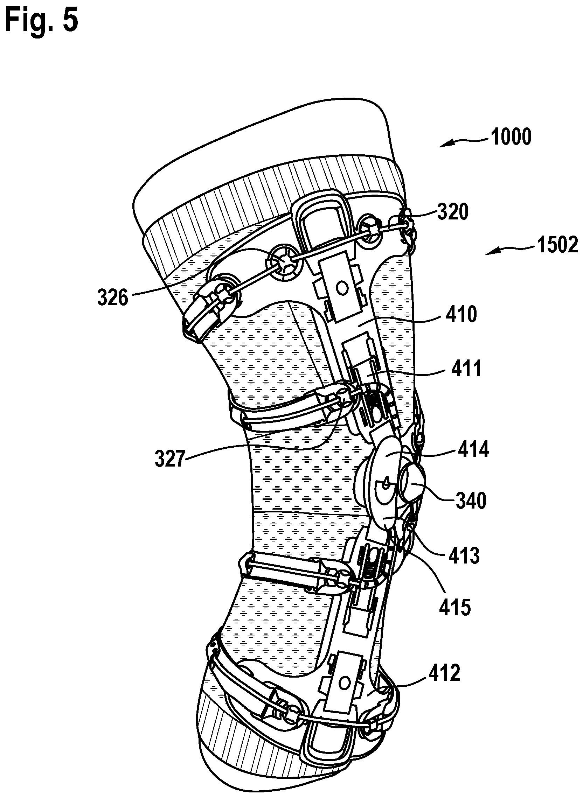

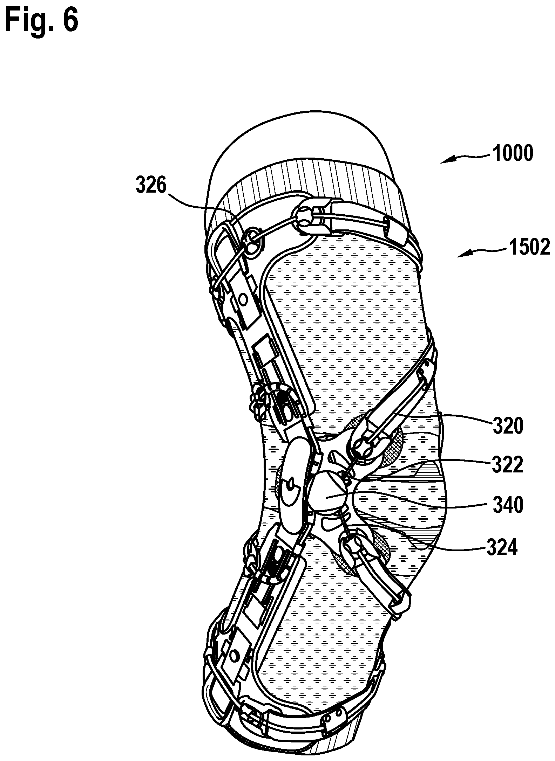

In Figur 5 und Figur 6 ist die Knieorthese (1000) an der Außenseite (1502) des Beins zu sehen. Hier verläuft eine erste Gelenkschiene (410) mit einem ersten Schienenabschnitt (411), der über ein erstes Gelenk (414) mit einem dritten Schienenabschnitt (413) verbunden ist und einem zweiten Schienenabschnitt (412), der auch über ein Gelenk (415) mit dem dritten Schienenabschnitt (413) verbunden ist. Am dritten Schienenabschnitt (413) befindet sich ein als Drehknopf (340) ausgebildetes Verbindungs- und Spannungsregelelement zum spannen des Seils des Bowdenzugs (320). Das erste Ende (322) und das zweite Ende (324) des Seils (323) enden am Drehknopf (340). Die Gelenkschiene (410) weist auch drehbar gelagerte Befestigungselemente (326) und drehbar gelagerte Umlenkführungen (327) für den Bowdenzug (320) auf.

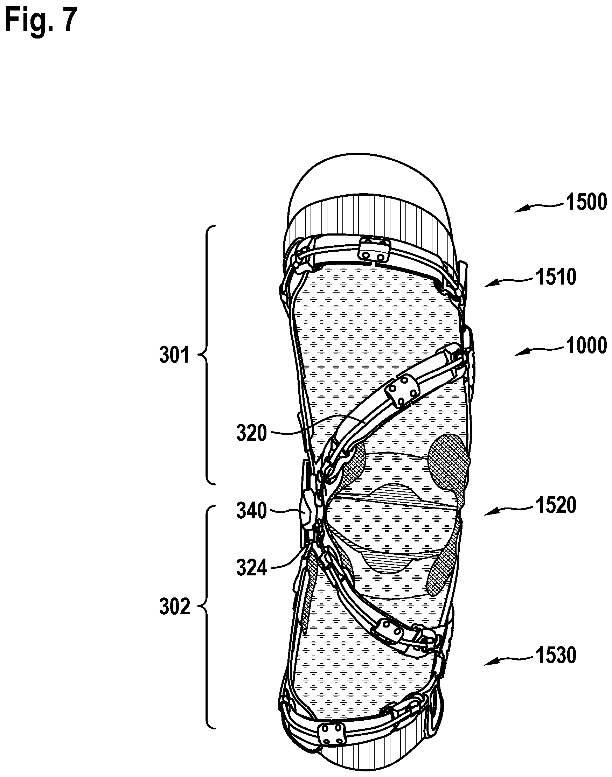

Figur 7 zeigt wieder wie Figur 1 die Vorderseite des Beins (1500) mit der Knieorthese (1000), wobei hier wieder die Aufteilung des durch den Bowdenzug (320) gebildeten Spannsystems (300) in ein erstes Spannteilsystem (301) am Oberschenkel (1510) und ein zweites Spannteilsystem (302) am Unterschenkel (1530) zu sehen ist, wobei der Drehknopf (340) als Spannungsregelelement im Kniebereich (1520) positioniert ist.

Die Figuren 1 bis 7 zeigen in der gegebenen Reihenfolge gut den Verlauf des Bowdenzugs (320) als Spannsystem (300):
Der Bowdenzug (320) als Spannelement (300) startet mit dem ersten Ende (324) in etwa auf Höhe des Knies am Spannungsregelelement (340) auf der Außenseite des Beins und verläuft nach oben über den Oberschenkel (1510) (Figur 1). Danach verläuft das Spannelement bis zu Innenseite (1501) des Beins und wird dort durch eine Führung (101) im ersten Kopplungselement (100) des ersten Kopplungssystems (10) des ersten Spannteilsystems (301) nach oben umgelenkt, worauf das Spannelement erneut über den Oberschenkel verläuft (Figur 2). Dann gleitet das Spannelement (300) über den vorderen Bereich des Oberschenkels zur Außenseite des Oberschenkels und verläuft über der Hinterseite des Oberschenkels zur Innenseite des Oberschenkels. Dort wird das Spannelement (300) durch eine Führung (201) im zweiten Kopplungselement (200) des ersten Kopplungssystems (10) nach unten umgelenkt und in Richtung hinterer Oberschenkel weitergeführt (Figur 3). Durch das geschlossene erste Kopplungssystem (10) kreuzt sich so das Spannelement (300) zweimalig. Das Spannelement (300) verläuft leicht schräg nach unten am hinteren Oberschenkel (Figur 4) zur Außenseite des Beins und wird dort senkrecht nach unten unter das Knie zum Unterschenkel und somit zum zweiten Spannteilsystem (302) geführt (Figur 5). Dort verläuft das Spannelement (300) über der Hinterseite des Unterschenkels zur Innenseite des Unterschenkels (Figur 4). Dort wird das Spannelement im durch eine Führung (201) zweiten Kopplungselement (200) des zweiten Kopplungssystems (10) des zweiten Spannteilsystems (302) nach unten umgelenkt und in Richtung hinterer Unterschenkel weitergeführt (Figur 3). Das Spannelement (300) verläuft am hinteren Unterschenkel zur Außenseite des Beins (Figur 5) und wird dort in Richtung vorderer Unterschenkel (Figur 6) und von da weiter zur Innenseite des Unterschenkels geführt (Figur 2). Dort wird das Spannelement (300) durch die Umlenkung (101) des ersten Kopplungselements (100) des zweiten Kopplungssystems (10) des zweiten Spannteilsystems (302) nach oben umgelenkt, worauf das Spannelement (300) erneut über die Vorderseite des Unterschenkels verläuft und mit dem zweiten Ende (324) wieder am Spannungsregelelement (340) auf der Außenseite endet (Figur 7). Mit dem Spannungsregelelement (340) können somit beide Enden (322,324) des Seils (323) des Bowdenzugs (320) gespannt werden.

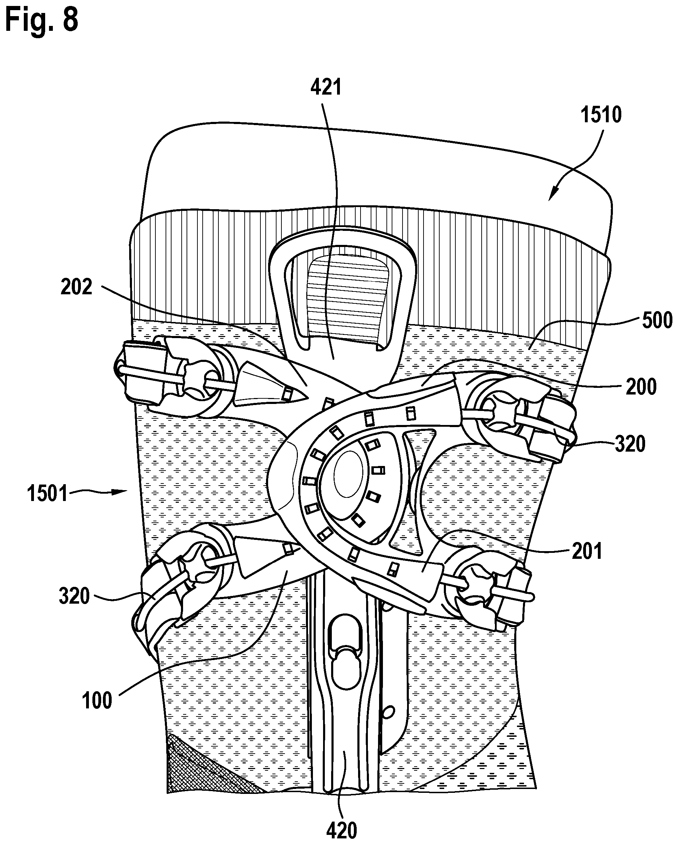

Figur 8 zeigt den Ausschnitt an der Innenseite (1501) des Oberschenkels (1510). Zu sehen ist das obere Kopplungssystem mit erstem Kopplungselement (100) mit Führung (101) für den Bowdenzug (320) und dem zweiten Kopplungselement (200) mit Führung (201) für den Bowdenzug (320). Die Bandage (500) aus einem Gestrick ist reversibel über eine Tasche mit dem oberen, ersten Schienenabschnitt (421) der zweiten Gelenkschiene (420) verbunden.

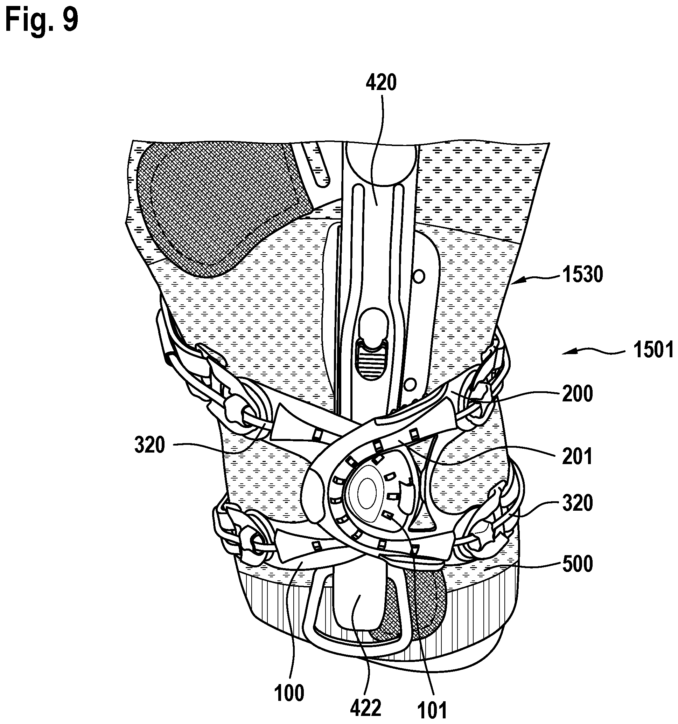

Figur 9 zeigt den Ausschnitt an der Innenseite (1501) des Unterschenkels (1530). Zu sehen ist das untere Kopplungssystem mit erstem Kopplungselement (100) mit Führung (101) für den Bowdenzug (320) und dem zweiten Kopplungselement (200) mit Führung (201) für den Bowdenzug (320). Die Bandage (500) aus einem Gestrick ist reversibel über eine Tasche mit dem unteren, zweiten Schienenabschnitt (422) der zweiten Gelenkschiene (420) verbindbar.

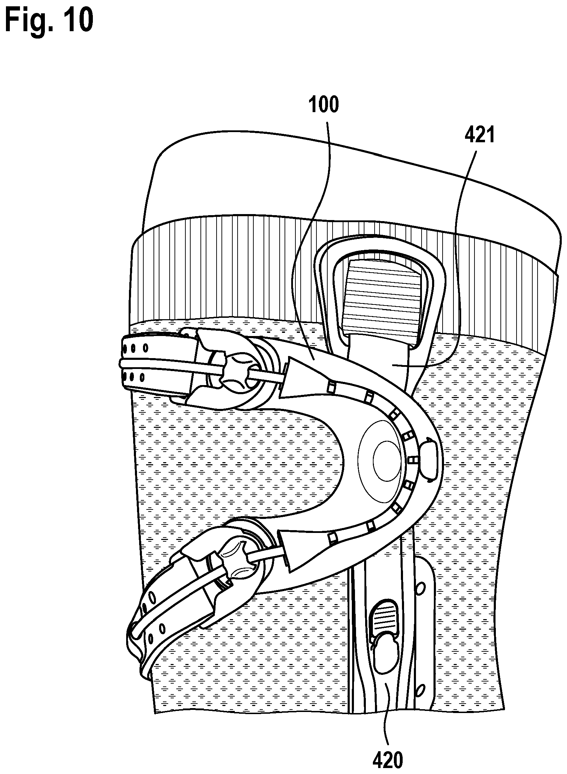

Figur 10 zeigt den Ausschnitt aus Figur 8 wobei das obere Kopplungssystem geöffnet ist und somit das erste Kopplungselement (100) von dem zweiten Kopplungselement getrennt ist. Das erste Kopplungselement (100) ist mit dem oberen, ersten Schienenabschnitt (421) der zweiten Gelenkschiene (420) verbunden.

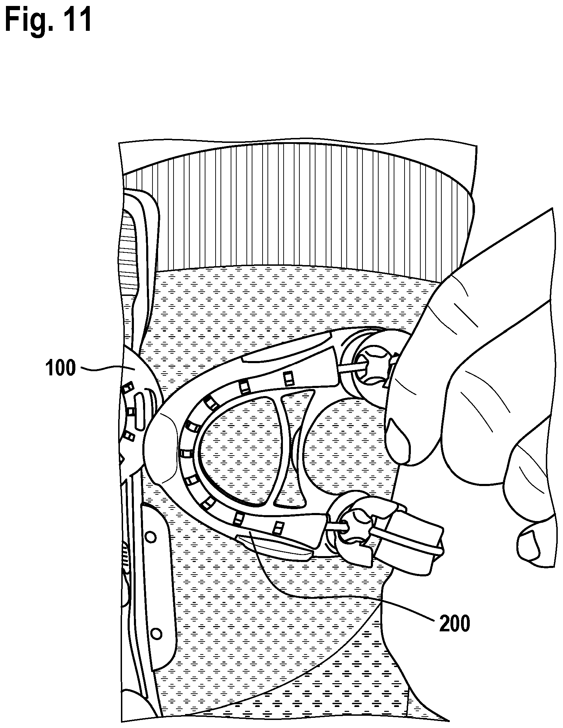

Figur 11 zeigt den Schließvorgang der offenen Knieorthese von Figur 10. Dabei wird das zweite Kopplungselement (200) zum ersten Kopplungselement (100) geführt und mit diesem verbunden.

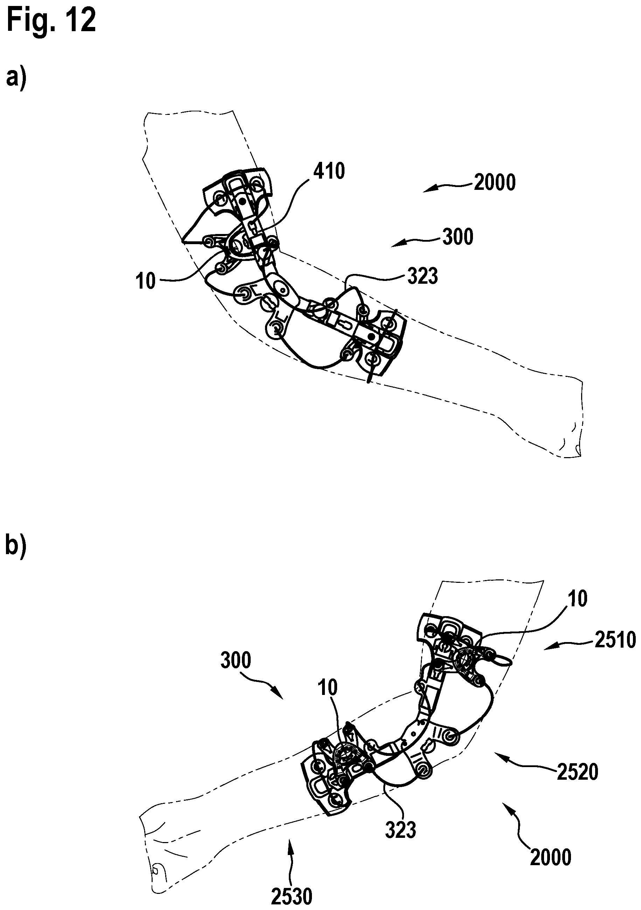

Figur 12 zeigt schematisch eine erfindungsgemäße Extremitätenorthese als Ellenbogenorthese (2000) an einem Arm (2500) mit Oberarm (2510), Ellenbogenbereich (2520) und Unterarm (2530). Der Ellenbogenorthese (2000) kann eine Bandage zugeordnet sein. Die Ellenbogenorthese umfasst ein Spannsystem (300) mit einem Seil (323). Figur 12 a) zeigt den Außenarm, Figur 12b zeigt den Innenarm. Am Außenarm liegt eine Gelenkschiene (410) an. Auf dem Innenarm liegen die beiden Kopplungssysteme (10) zum Öffnen und Schließen der Ellenbogenorthese (2000). Details der Ausführung können von der Knieorthese (1000) aus den Figuren 1 bis 7 übernommen werden.

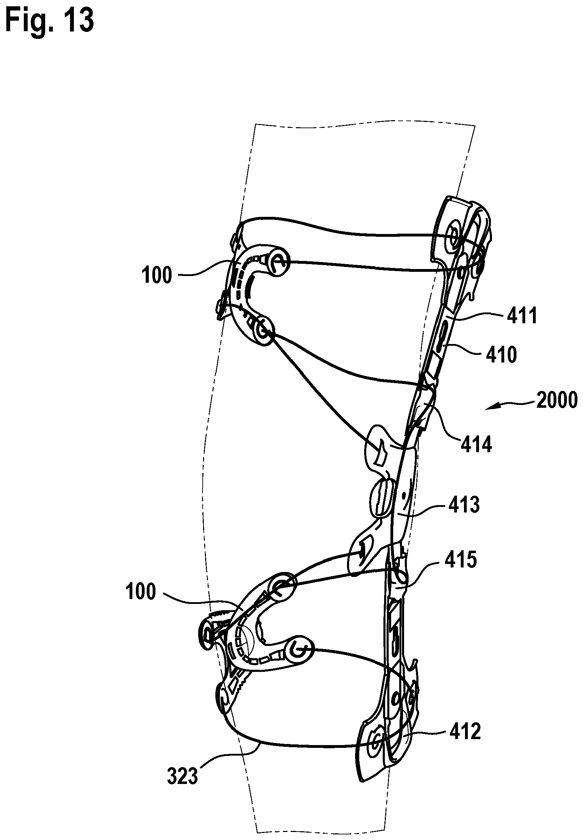

Figur 13 zeigt schematisch die Ellenbogenorthese (2000) mit Seil (323) und Kopplungssystemen (100) aus Figur 12 aus anderer Perspektive. Die Gelenkschiene (410) hat einen ersten Schienenabschnitt (411), der über ein erstes Gelenk (414) mit einem dritten Schienenabschnitt (413) verbunden ist und einen zweiten Schienenabschnitt (412), der auch über ein Gelenk (415) mit dem dritten Schienenabschnitt (413) verbunden ist. Am dritten Schienenabschnitt (413) befindet sich ein nicht gezeigtes Spannungsregelelement.

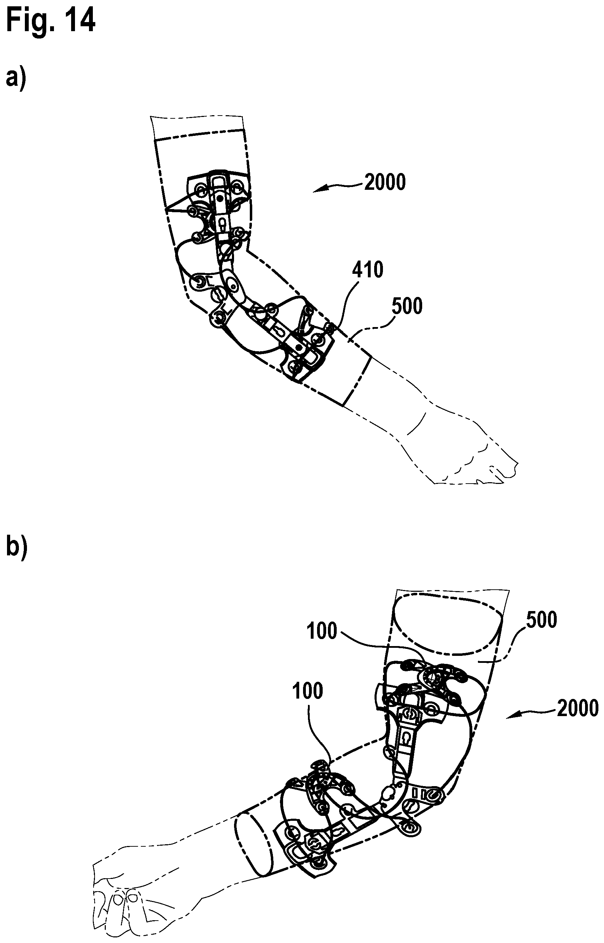

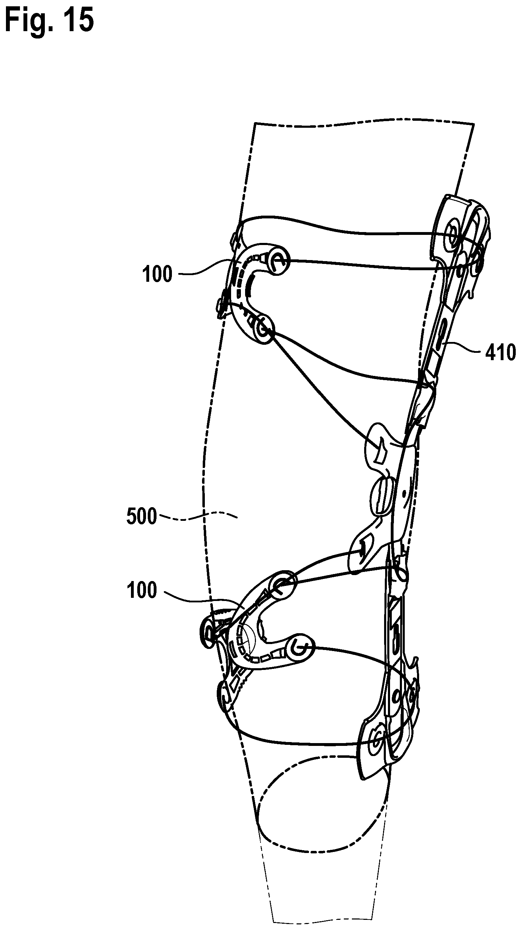

Figur 14a, Figur 14b und Figur 15 zeigt eine andere Ausführungsform der Ellenbogenorthese (2000) mit Kopplungssystemen (100) und Gelenkschiene (410) aus den Figuren 12 und 13, bei der der Ellenbogenorthese (2000) eine Bandage (500) zugeordnet ist. Der Aufbau entspricht ansonsten dem der Figuren 12 und 13.

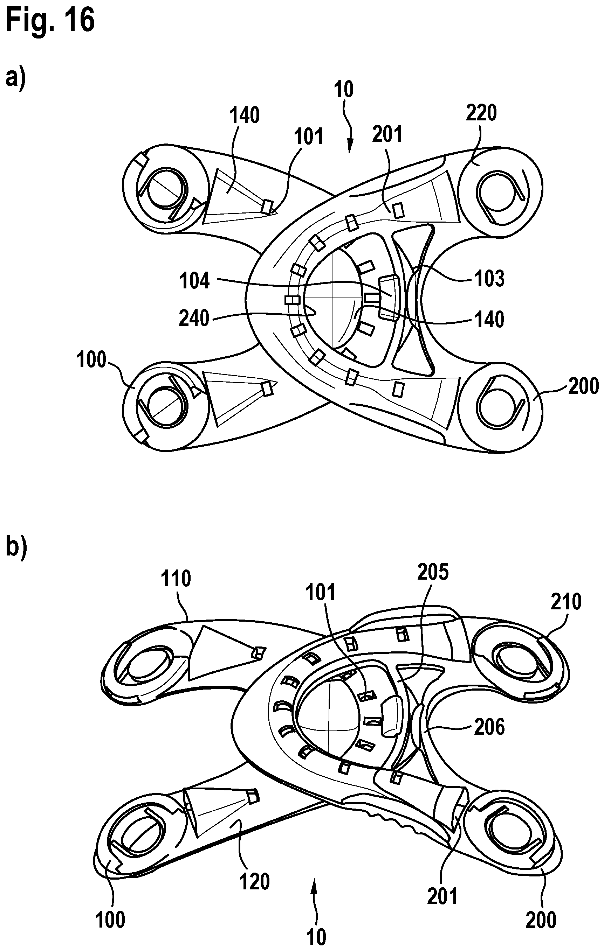

Figur 16 zeigt die Vorderseite einer bevorzugten Ausführungsform einer Kopplungsvorrichtung (10) für eine erfindungsgemäße Orthese mit dem ersten Kopplungselement (100) und dem zweiten Kopplungselement (200) in Draufsicht (Figur 16a) und in Schrägansicht (Figur 16b) und in geschlossenem Zustand. Zu sehen sind dementsprechend die Vorderseite (120) des ersten Kopplungselements (100) und die Vorderseite (220) des zweiten Kopplungselements (200). Beide Kopplungselemente (100,200) sind hufeisenförmig und haben dementsprechend einen äußeren Randbereich (110,210). Die erste Führung (101) für ein Spannelement folgt der Hufeisenform des ersten Kopplungselements (100) und die zweite Führung (201) für ein Spannelement folgt der Hufeisenform des ersten Kopplungselements (200). Dabei ist die zweite Führung (201) aus der Vorderseite (220) des zweiten Kopplungselements (200) heraus gewölbt und die erste Führung (101) ist auf der Vorderseite (120) des ersten Kopplungselements (100) flach, so dass die Vorderseite (120) des ersten Kopplungselements (100) auf der Rückseite des zweiten Kopplungselements (200) flach aufliegen kann. In einer alternativen Ausführungsform kann auf die Führungen (101,201) auch verzichtet werden, insbesondere wenn kein Spannelement vorgesehen ist. Das erste Kopplungselement (100) hat an seiner Spitze (103) der Hufeisenform ein erstes Rastelement (104), in das das erste gekrümmte Stegelement (205) des zweiten Kopplungselements (200), das den konkaven Innenbereich der Hufeisenform des zweiten Kopplungselements (200) überspannt, eingerastet ist. Das zweite in die andere Richtung gekrümmte Stegelement (206) liegt nahe bei dem ersten Stegelement (205), so dass das erste Stegelement (205) zwischen dem ersten Rastelement (104) und dem zweiten Stegelement (206) eingeklemmt ist. Sowohl das erste Kopplungselement (100) weist Griffelemente (140) auf als auch das zweite Kopplungselement (200) weist Griffelemente (240) auf, die in vorteilhafter Weise aus den kanalartigen Führungen (101,201) gebildet werden.

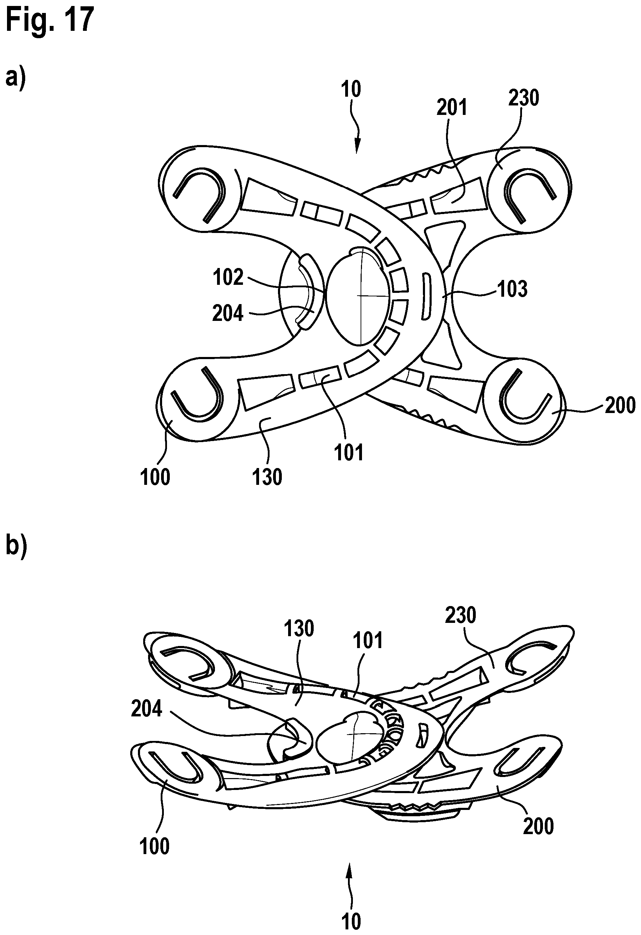

Figur 17 zeigt die Rückseite der Kopplungsvorrichtung (10) aus Figur 16 mit dem ersten Kopplungselement (100) und dem zweiten Kopplungselement (200) in Draufsicht (Figur 2a) und in Schrägansicht (Figur 2b) und in geschlossenem Zustand. Zu sehen sind dementsprechend die Rückseite (130) des ersten Kopplungselements (100) und die Rückseite (230) des zweiten Kopplungselements (200). Die erste Führung (101) für ein Spannelement ist als Kanal mit Durchbrechungen ausgeführt und folgt der Hufeisenform des ersten Kopplungselements (100) und die zweite Führung (201) für ein Spannelement ist ebenfalls als Kanal mit Durchbrechungen ausgeführt und folgt der Hufeisenform des ersten Kopplungselements (200). Dabei ist die erste Führung (101) aus der Rückseite (130) heraus gewölbt und die zweite Führung (201) ist auf der Rückseite (230) des zweiten Kopplungselements (200) flach, so dass die Vorderseite des ersten Kopplungselements (100) auf der Rückseite (230) des zweiten Kopplungselements (200) flach aufliegen kann. Das erste hufeisenförmige Kopplungselement (100) hat eine konkave Innenkante (102) und außen eine Spitze (103). Die konkave Innenkante (102) des ersten Kopplungselements (100) ist in ein zweites Rastelement (204) auf der Rückseite (230) des zweiten Kopplungselements (200) eingerastet.

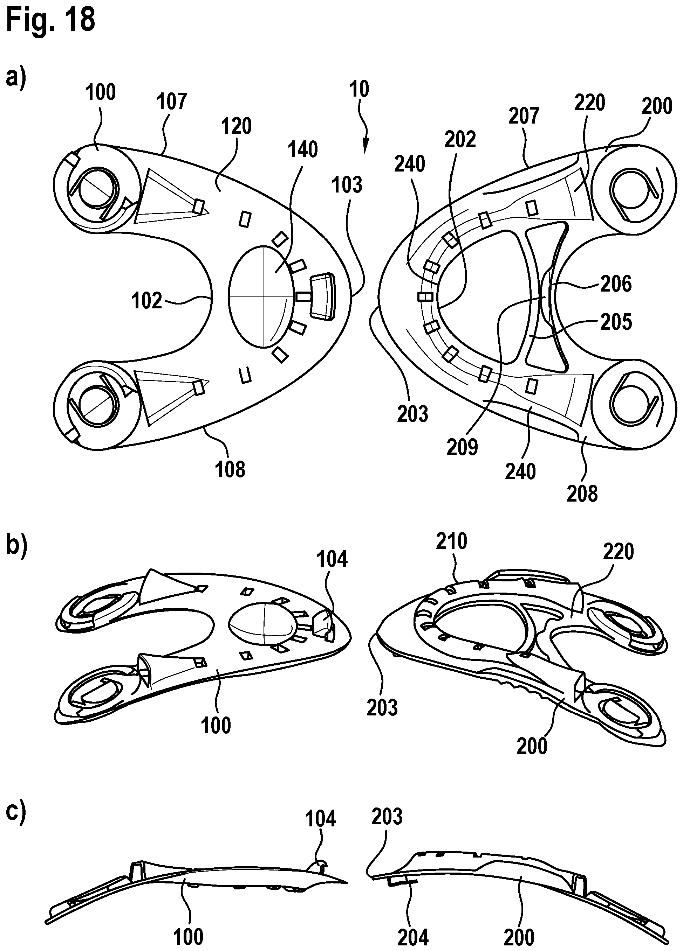

Figur 18 zeigt die Vorderseite einer Kopplungsvorrichtung (10) aus Figur 16 mit dem ersten Kopplungselement (100) und dem zweiten Kopplungselement (200) in Draufsicht (Figur 3a) und in Schrägansicht (Figur 3b) und in Seitenansicht (Figur 3c) in geöffnetem Zustand. Zu sehen sind dementsprechend die Vorderseite (120) des ersten Kopplungselements (100) und die Vorderseite (220) des zweiten Kopplungselements (200). Beide Kopplungselemente (100,200) sind hufeisenförmig und haben dementsprechend einen äußeren Randbereich (210) sowie einen ersten Arm (107,207) und einen zweiten Arm (108,208).

Das erste Kopplungselement (100) hat eine konkave Innenkante (102) und an seiner Spitze (103) der Hufeisenform ein erstes Rastelement (104), in das das erste gekrümmte Stegelement (205) des zweiten Kopplungselements (200), das den konkaven Innenbereich der Hufeisenform des zweiten Kopplungselements (200) überspannt, einrastbar ist. Das zweite in die andere Richtung gekrümmte Stegelement (206) liegt nahe bei dem ersten Stegelement (205), so dass das erste Stegelement (205) zwischen dem ersten Rastelement (104) und dem zweiten Stegelement (206) einklemmbar ist. Das Stegelement (206) hat in Richtung des ersten Stegelements (205) eine schräge Abflachung (209), die sich unter das erste Stegelement (205) schieben kann. Das zweite Kopplungselement (200) hat eine konkave Innenkante (202) und an seiner Spitze (203) der Hufeisenform auf der Unterseite ein zweites Rastelement (204), in das die konkave Innenkante (102) des ersten Kopplungselements (100) einrastbar ist. Sowohl das erste Kopplungselement (100) als auch das zweite Kopplungselement (200) weist Griffelemente (140, 240) auf.

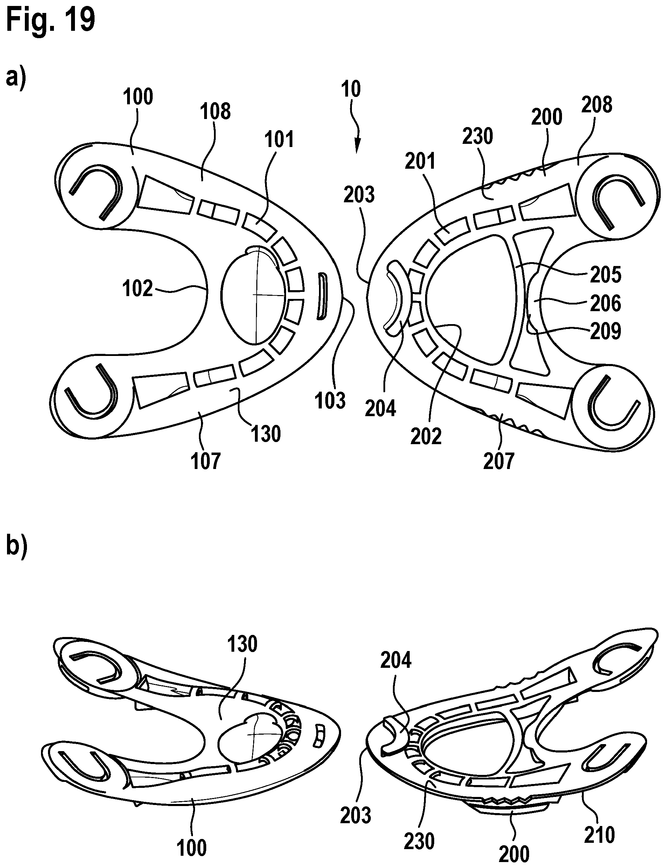

Figur 19 zeigt die Rückseite der Kopplungsvorrichtung (10) aus Figur 16 mit dem ersten hufeisenförmigen Kopplungselement (100) mit erstem (107) und zweitem (108) Arm und dem zweiten hufeisenförmigen Kopplungselement (200) mit erstem (207) und zweitem (208) Arm und dem Randbereich (210) in Draufsicht (Figur 4a) und in Schrägansicht (Figur 4b) und in geöffnetem Zustand. Zu sehen sind dementsprechend die Rückseite (130) des ersten Kopplungselements (100) und die Rückseite (230) des zweiten Kopplungselements (200). Die erste Führung (101) für ein Spannelement ist als Kanal mit Durchbrechungen ausgeführt und folgt der Hufeisenform des ersten Kopplungselements (100) und die zweite Führung (201) für ein Spannelement ist ebenfalls als Kanal mit Durchbrechungen ausgeführt und folgt der Hufeisenform des ersten Kopplungselements (200). Dabei ist die erste Führung (101) aus der Rückseite (130) heraus gewölbt und die zweite Führung (201) ist auf der Rückseite (230) des zweiten Kopplungselements (200) flach, so dass die Vorderseite des ersten Kopplungselements (100) auf der Rückseite (230) des zweiten Kopplungselements (200) flach aufliegen kann. Das erste hufeisenförmige Kopplungselement (100) hat eine konkave Innenkante (102) und außen eine Spitze (103). Die konkave Innenkante (102) des ersten Kopplungselements (100) kann in ein zweites Rastelement (204) auf der Rückseite (230) an der Spitze (203) des zweiten Kopplungselements (200) eingerastet werden. Das zweite Kopplungselement (200) hat eine konkave Innenkante (202), deren Zwischenraum von dem ersten Stegelement (205) und dem zweiten Stegelement (206) mit der schrägen Abflachung (209) überspannt wird.

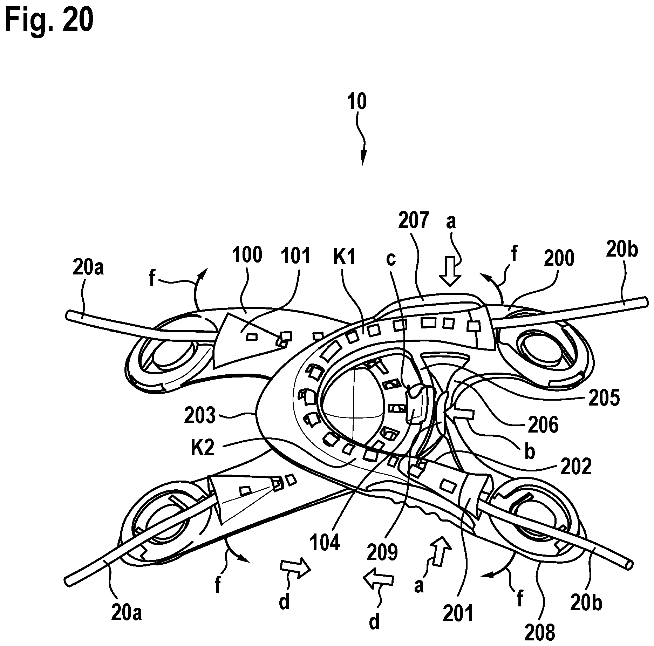

Figur 20 zeigt die Kopplungsvorrichtung (10) aus Figur 16 mit dem ersten Kopplungselement (100) und dem zweiten Kopplungselement (200) schematisch während der Verwendung, insbesondere vor/bei dem Öffnen der Kopplungsvorrichtung (10). In der ersten Führung (101) verläuft ein erstes Spannelement (20a), beispielsweise ein Seil oder ein Bowdenzug und in der zweiten Führung (201) verläuft ein zweites Spannelement (20b), beispielsweise ebenfalls ein Seil oder ein Bowdenzug. Bevorzugt kann es sich bei dem ersten Spannelement (20a) und dem zweiten Spannelement (20b) um Teilabschnitte eines einzigen Spannelements handeln. Sowohl die Führungen (101, 201) als auch die Spannelemente (20a, 20b) kreuzen sich im geschlossenen Zustand der Kopplungsvorrichtung (10) in vorteilhafter Weise zweimal, nämlich an einem ersten Kreuzungspunkt (K1) und an einem zweiten Kreuzungspunkt (K2). Ein Vorteil der erfindungsgemäßen Kopplungsvorrichtung (10) ist, dass die beiden Kopplungselemente (100, 200) gegeneinander drehbar gelagert sind, wie es durch die Bewegungsrichtungen (f) angedeutet ist.

Die Kopplungsvorrichtung (10) wird dadurch geöffnet, dass das zweite hufeisenförmige Kopplungselement (200) an den Armen (207, 208) zusammengedrückt wird, wie durch die Pfeile (a) angedeutet, wodurch das zweite die konkave Innenkante (202) des zweiten Kopplungselements (200) überspannendes Stegelement (206) mit seiner schrägen Abflachung (209) in Richtung Spitze (203) des zweiten hufeisenförmigen Kopplungselements (200) und in Richtung des ersten Kopplungselements (100) geschoben wird, wie durch den Pfeil (b) angedeutet, und wodurch das zweite die konkave Innenkante (202) des zweiten Kopplungselements (200) überspannendes Stegelement (206) mit seiner schrägen Abflachung (209) das erste Stegelement (205) aus dem ersten Rastelement (104) heraushebt, wie durch den Pfeil (c) angedeutet, so dass die beiden Kopplungselemente (100, 200) gegeneinander geschoben werden und dadurch voneinander getrennt werden können, wie durch die Pfeile (d) angedeutet.

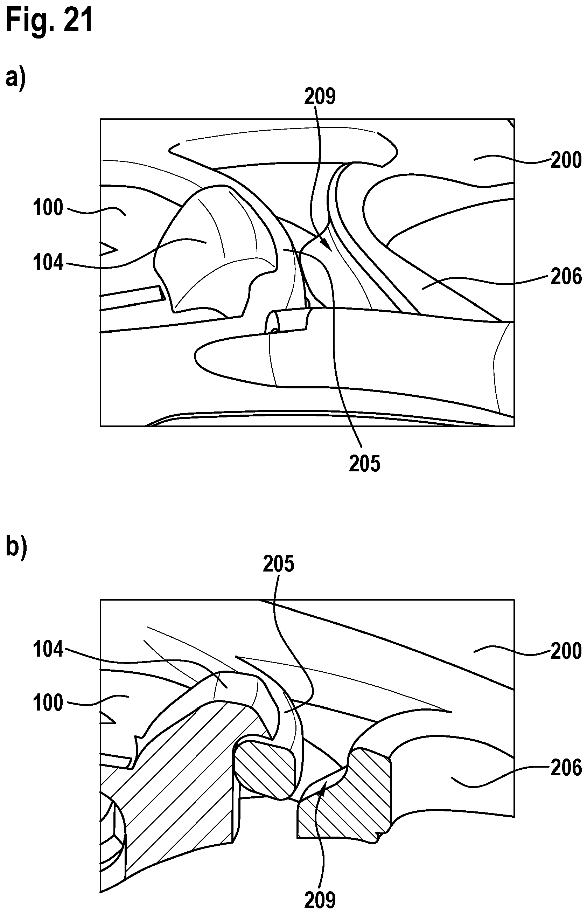

Figur 21 zeigt zwei Detailansichten der Vorderseite der Kopplungsvorrichtung aus Figur 16 mit dem ersten Kopplungselement (100) und dem zweiten Kopplungselement (200) in Schrägansicht (Figur 21a) und im Querschnitt (Figur 21b) im geschlossenen Zustand. Das erste Kopplungselement (100) hat an seiner Spitze ein erstes Rastelement (104), in das das erste gekrümmte Stegelement (205) des zweiten Kopplungselements (200), das den konkaven Innenbereich der Hufeisenform des zweiten Kopplungselements (200) überspannt, eingerastet ist. Das zweite in die andere Richtung gekrümmte Stegelement (206) liegt nahe bei dem ersten Stegelement (205), so dass das erste Stegelement (205) zwischen dem ersten Rastelement (104) und dem zweiten Stegelement (206) eingeklemmt ist. Das Stegelement (206) hat in Richtung des ersten Stegelements (205) eine schräge Abflachung (209), die sich unter das erste Stegelement (205) schieben kann.

### BEZUGSZEICHENLISTE

- 10: Kopplungsvorrichtung
- 20a: Spannelement
- 20b: Spannelement
- 100: erstes Kopplungselement
- 101: erste Führung
- 102: konkave Innenkante des ersten Kopplungselements
- 103: Spitze der Hufeisenform des ersten Kopplungselements
- 104: erstes Rastelement des ersten Kopplungselements
- 107: erster Arm des hufeisenförmigen ersten Kopplungselements
- 108: zweiter Arm des hufeisenförmigen ersten Kopplungselements
- 110: Randbereich des ersten Kopplungselements
- 120: Vorderseite des ersten Kopplungselements
- 130: Rückseite des ersten Kopplungselements
- 140: Griffelement des ersten Kopplungselements
- 200: zweites Kopplungselement
- 201: zweite Führung
- 202: konkave Innenkante des zweiten Kopplungselements
- 203: Spitze der Hufeisenform des zweiten Kopplungselements
- 204: zweites Rastelement des zweiten Kopplungselements
- 205: erstes Stegelement
- 206: zweites Stegelement
- 207: erster Arm des hufeisenförmigen zweiten Kopplungselements
- 208: zweiter Arm des hufeisenförmigen zweiten Kopplungselements
- 209: schräge Abflachung am zweiten Stegelement
- 210: Randbereich des zweiten Kopplungselements
- 220: Vorderseite des zweiten Kopplungselements
- 230: Rückseite des zweiten Kopplungselements
- 240: Griffelement des zweiten Kopplungselements
- 300: Spannsystem
- 301: erstes Spannteilsystem
- 302: zweites Spannteilsystem
- 320: Bowdenzug als Spannelement
- 321: Ummantelung
- 322: erstes Ende des Seils des Bowdenzugs
- 323: Seil
- 324: zweites Ende des Seils des Bowdenzugs
- 325: Polsterung
- 326: drehbar gelagertes Befestigungselement
- 327: drehbar gelagerte Umlenkführung
- 340: Drehknopf als Verbindungs- und Spannungsregelelement
- 410: erste Gelenkschiene
- 411: erster Schienenabschnitt
- 412: zweiter Schienenabschnitt
- 413: dritter Schienenabschnitt
- 414: erstes Gelenk
- 415: zweites Gelenk
- 420: zweite Gelenkschiene
- 421: erster Schienenabschnitt
- 422: zweiter Schienenabschnitt
- 423: dritter Schienenabschnitt
- 424: erstes Gelenk
- 425: zweites Gelenk
- 500: Bandage
- 1000: Knieorthese
- 1500: Bein
- 1501: Innenseite des Beins
- 1502: Außenseite des Beins
- 1510: Oberschenkel
- 1520: Kniebereich
- 1530: Unterschenkel
- 2000: Ellenbogenorthese
- 2500: Arm
- 2501: Außenseite des Arms
- 2502: Innenseite des Arms
- 2510: Oberarm
- 2520: Ellenbogenbereich
- 2530: Unterarm
- a: erster Schritt des Öffnens
- b: zweiter Schritt des Öffnens
- c: dritter Schritt des Öffnens
- d: vierter Schritt des Öffnens
- f: Drehbewegung
- K1: erster Kreuzungspunkt
- K2: zweiter Kreuzungspunkt

## Patentansprüche

1. Extremitätenorthese, Extremitätenbandage oder Sporthilfsmittel, umfassend ein Spannsystem (300) aus einem ersten Spannteilsystem (301) und einem zweiten Spannteilsystem (302), **dadurch gekennzeichnet, dass** das erste Spannteilsystem (301) und das zweite Spannteilsystem (302) jeweils durch eine Kopplungsvorrichtung reversibel schließbar sind, wobei jede Kopplungsvorrichtung ein erstes Kopplungselement (100) und ein zweites Kopplungselement (200) umfasst, wobei das erste Kopplungselement (100) mit dem zweiten Kopplungselement (200) über ein Rastsystem reversibel miteinander verbindbar sind, wobei das erste Kopplungselement (100) eine erste Führung (101) für ein Spannelement (20a) aufweist und das zweite Kopplungselement (200) eine zweite Führung (201) für ein Spannelement (20b) aufweist, wobei das Spannelement (20a) in der ersten Führung (101) verläuft und das Spannelement (20b) in der zweiten Führung (201) verläuft und wobei im verbundenen Zustand der beiden Kopplungselemente (100, 200) sich die erste Führung (101) und die zweite Führung (201) zweimal kreuzen.

2. Extremitätenorthese, Extremitätenbandage oder Sporthilfsmittel nach Anspruch 1 **dadurch gekennzeichnet, dass** das erste Spannteilsystem (301) und das zweite Spannteilsystem (302) aus einem durchgehenden Spannelement gebildet werden.

3. Extremitätenorthese, Extremitätenbandage oder Sporthilfsmittel nach Anspruch 1 **dadurch gekennzeichnet, dass** das erste Spannteilsystem (301) aus einem ersten Spannelement gebildet wird und das zweite Spannteilsystem (302) aus einem zweiten Spannelement gebildet wird.

4. Extremitätenorthese, Extremitätenbandage oder Sporthilfsmittel nach Anspruch 2 oder nach Anspruch 3, **dadurch gekennzeichnet, dass** das mindestens eine Spannelement ein Seil, ein Draht, ein Flachband oder ein Bowdenzug (320) ist.

5. Extremitätenorthese, Extremitätenbandage oder Sporthilfsmittel nach Anspruch 4, **dadurch gekennzeichnet, dass** das mindestens eine Spannelement ein Seil oder einen Draht umfasst, wobei das Seil oder der Draht zumindest bereichsweise in einer Ummantelung (321) verläuft, wobei die Ummantelung an der Extremitätenorthese verschiebbar gelagert ist.

6. Extremitätenorthese, Extremitätenbandage oder Sporthilfsmittel nach einem der vorhergehenden Ansprüche, wobei das erste Kopplungselement (100) eine Vorderseite (120) und eine Rückseite (130) aufweist und eine Hufeisenform mit einer konkaven Innenkante (102) aufweist und wobei das zweite Kopplungselement (200) eine Vorderseite (220) und eine Rückseite (230) aufweist und eine Hufeisenform mit einer konkaven Innenkante (202) aufweist, wobei das erste Kopplungselement (100) im Bereich der Spitze (103) der Hufeisenform auf der Vorderseite (120) ein erstes Rastelement (104) aufweist und das zweite Kopplungselement (200) im Bereich der Spitze (203) der Hufeisenform auf der Rückseite (230) ein zweites Rastelement (204) aufweist und wobei das zweite hufeisenförmige Kopplungselement (200) ein die konkave Innenkante (202) des zweiten Kopplungselements (200) überspannendes erstes Stegelement (205) aufweist, wobei im verbundenen Zustand der beiden Kopplungselemente (100, 200) das erste Rastelement (104) des ersten Kopplungselements (100) in das erste Stegelement (205) des zweiten Kopplungselements (200) eingreift und das zweite Rastelement (204) des zweiten Kopplungselements (200) in die konkave Innenkante (102) des ersten Kopplungselements (100) eingreift.

7. Extremitätenorthese, Extremitätenbandage oder Sporthilfsmittel nach einem der vorhergehenden Ansprüche, **dadurch gekennzeichnet, dass** die Extremitätenorthese drehbar gelagerte Umlenkungsführungen (327) aufweist, in denen das mindestens eine Spannelement umgelenkt wird.

8. Extremitätenorthese, Extremitätenbandage oder Sporthilfsmittel nach einem der vorhergehenden Ansprüche, **dadurch gekennzeichnet, dass** die Extremitätenorthese oder -bandage eine Knieorthese (1000) oder -bandage ist oder das Sporthilfsmittel ein Sporthilfsmittel für das Knie.

9. Extremitätenorthese, Extremitätenbandage oder Sporthilfsmittel nach einem der vorhergehenden Ansprüche, **dadurch gekennzeichnet, dass** bei der angelegten Extremitätenorthese das erste Spannteilsystem (301) und/oder das zweite Spannteilsystem (302) des Spannsystems (300) so um die Extremität, insbesondere ein Bein (1500), geführt sind, dass das erste Spannteilsystem (301) und/oder das zweite Spannteilsystem (302) des Spannsystems (300) jeweils die Extremität umschließt.

10. Extremitätenorthese, Extremitätenbandage oder Sporthilfsmittel nach einem der vorhergehenden Ansprüche, **dadurch gekennzeichnet, dass** die Extremitätenorthese ein Verbindungselement aufweist, an dem das mindestens eine Spannelement, insbesondere das eine Spannelement nach Anspruch 2 oder das erste Spannelement und/oder das zweite Spannelement nach Anspruch 3 befestigt sind.

11. Extremitätenorthese, Extremitätenbandage oder Sporthilfsmittel nach Anspruch 10, **dadurch gekennzeichnet, dass** das eine Spannelement nach Anspruch 2 mit beiden Enden am Verbindungselement befestigt ist.

12. Extremitätenorthese, Extremitätenbandage oder Sporthilfsmittel nach Anspruch 10 oder Anspruch 11, **dadurch gekennzeichnet, dass** das Verbindungselement ein Spannungsregelelement (340) zum Spannen des mindestens einen Spannelements aufweist.

13. Extremitätenorthese, Extremitätenbandage oder Sporthilfsmittel nach einem der vorhergehenden Ansprüche, **dadurch gekennzeichnet, dass** die Extremitätenorthese, Extremitätenbandage oder Sporthilfsmittel mindestens eine Gelenkschiene (410), bevorzugt zwei Gelenkschienen (410,420) aufweist.

14. Extremitätenorthese, Extremitätenbandage oder Sporthilfsmittel nach Anspruch 13, **dadurch gekennzeichnet, dass** die mindestens eine Gelenkschiene (410) einen ersten Schienenabschnitt (411), einen zweiten Schienenabschnitt (412) und einen dritten Schienenabschnitt aufweist (413), wobei der erste Schienenabschnitt (411) und der zweite Schienenabschnitt (412) jeweils durch ein Gelenk (414,415) mit dem dritten Schienenabschnitt (413) verbunden sind.

15. Extremitätenorthese, Extremitätenbandage oder Sporthilfsmittel nach Anspruch 14, **dadurch gekennzeichnet, dass** das Verbindungselement (340) nach einem der Ansprüche 10 bis 12 am dritten Schienenabschnitt (413) der mindestens einen Gelenkschiene (410) befestigt ist.

## Claims

1. Limb orthosis, limb bandage or sports aid device, comprising a tensioning system (300) consisting of a first tensioning sub-system (301) and a second tensioning sub-system (302), **characterized in that** the first tensioning sub-system (301) and the second tensioning sub-system (302) are each reversibly closable by a coupling device, wherein each coupling device comprises a first coupling element (100) and a second coupling element (200), wherein the first coupling element (100) is reversibly connectable to the second coupling element (200) via a latching system, wherein the first coupling element (100) comprises a first guide (101) for a tensioning element (20a) and the second coupling element (200) comprises a second guide (201) for a tensioning element (20b), wherein the tensioning element (20a) runs in the first guide (101) and the tensioning element (20b) runs in the second guide (201) and wherein, in the connected state of the two coupling elements (100, 200), the first guide (101) and the second guide (201) cross each other twice.

2. Limb orthosis, limb bandage or sports aid device according to claim 1, **characterized in that** the first tensioning sub-system (301) and the second tensioning sub-system (302) are formed from a continuous tensioning element.

3. Limb orthosis, limb bandage or sports aid device according to claim 1, **characterized in that** the first tensioning sub-system (301) is formed from a first tensioning element and the second tensioning sub-system (302) is formed from a second tensioning element.

4. Limb orthosis, limb bandage or sports aid device according to claim 2 or claim 3, **characterized in that** the at least one tensioning element is a rope, a wire, a flat band or a Bowden cable (320).

5. Limb orthosis, limb bandage or sports aid device according to claim 4, **characterized in that** the at least one tensioning element comprises a rope or a wire, wherein the rope or the wire runs at least partially in a sheathing (321), wherein the sheathing is displaceably mounted on the limb orthosis.

6. Limb orthosis, limb bandage or sports aid device according to one of the preceding claims, wherein the first coupling element (100) comprises a front side (120) and a back side (130) and comprises a horseshoe shape with a concave inner edge (102), and wherein the second coupling element (200) comprises a front side (220) and a back side (230) and comprises a horseshoe shape with a concave inner edge (202), wherein the first coupling element (100) comprises a first latching element (104) in the area of the tip (103) of the horseshoe shape on the front side (120) and the second coupling element (200) comprises a second latching element (204) in the area of the tip (203) of the horseshoe shape on the back side (230) and wherein the second horseshoe-shaped coupling element (200) comprises a first bar element (205) spanning the concave inner edge (202) of the second coupling element (200), wherein, in the connected state of the two coupling elements (100, 200), the first latching element (104) of the first coupling element (100) engages in the first bar element (205) of the second coupling element (200) and the second latching element (204) of the second coupling element (200) engages in the concave inner edge (102) of the first coupling element (100).

7. Limb orthosis, limb bandage or sports aid device according to one of the preceding claims, **characterized in that** the limb orthosis comprises rotatably mounted deflection guides (327) in which the at least one tensioning element is deflected.

8. Limb orthosis, limb bandage or sports aid device according to one of the preceding claims, **characterized in that** the limb orthosis or bandage is a knee orthosis (1000) or bandage or the sports aid device is a sports aid device for the knee.

9. Limb orthosis, limb bandage or sports aid device according to one of the preceding claims, **characterized in that**, when the limb orthosis is applied, the first tensioning sub-system (301) and/or the second tensioning sub-system (302) of the tensioning system (300) are guided around the limb, in particular a leg (1500), in such a way that the first tensioning sub-system (301) and/or the second tensioning sub-system (302) of the tensioning system (300) each enclose the limb.

10. Limb orthosis, limb bandage or sports aid device according to one of the preceding claims, **characterized in that** the limb orthosis comprises a connection element to which the at least one tensioning element, in particular the single tensioning element according to claim 2 or the first tensioning element and/or the second tensioning element according to claim 3, are fastened.

11. Limb orthosis, limb bandage or sports aid device according to claim 10, **characterized in that** the tensioning element according to claim 2 is fastened with both ends to the connection element.

12. Limb orthosis, limb bandage or sports aid device according to claim 10 or claim 11, **characterized in that** the connection element comprises a tension control element (340) for tensioning the at least one tensioning element.

13. Limb orthosis, limb bandage or sports aid device according to one of the preceding claims, **characterized in that** the limb orthosis, limb bandage or sports aid device comprises at least one joint splint (410), preferably two joint splints (410, 420).

14. Limb orthosis, limb bandage or sports aid device according to claim 13, **characterized in that** the at least one joint splint (410) comprises a first splint section (411), a second splint section (412) and a third splint section (413), wherein the first splint section (411) and the second splint section (412) are each connected to the third splint section (413) by a joint (414, 415).

15. Limb orthosis, limb bandage or sports aid device according to claim 14, **characterized in that** the connection element (340) according to one of the claims 10 to 12 is fastened to the third splint section (413) of the at least one joint splint (410).

## Revendications

1. Orthèse de membre, bandage de membre ou accessoire de sport, comportant un système de tension (300) constitué d'un premier sous-système de tension (301) et d'un deuxième sous-système de tension (302), **caractérisé en ce que** le premier sous-système de tension (301) et le deuxième sous-système de tension (302) peuvent être fermés de manière réversible respectivement par le biais d'un dispositif d'accouplement, dans lequel chaque dispositif d'accouplement comporte un premier élément d'accouplement (100) et un deuxième élément d'accouplement (200), dans lequel le premier élément d'accouplement (100) et le deuxième élément d'accouplement (200) peuvent être reliés l'un à l'autre de manière réversible par l'intermédiaire d'un système d'encliquetage, dans lequel le premier élément d'accouplement (100) comprend un premier guide (101) pour un élément de tension (20a) et le deuxième élément d'accouplement (200) comprend un deuxième guide (201) pour un élément de tension (20b), dans lequel l'élément de tension (20a) s'étend dans le premier guide (101) et l'élément de tension (20b) s'étend dans le deuxième guide (201) et dans lequel, dans l'état relié des deux éléments d'accouplement (100, 200), le premier guide (101) et le deuxième guide (201) se croisent deux fois.

2. Orthèse de membre, bandage de membre ou accessoire de sport selon la revendication 1, **caractérisé en ce que** le premier sous-système de tension (301) et le deuxième sous-système de tension (302) sont formés à partir d'un élément de tension continu.

3. Orthèse de membre, bandage de membre ou accessoire de sport selon la revendication 1, **caractérisé en ce que** le premier sous-système de tension (301) est formé à partir d'un premier élément de tension et le deuxième sous-système de tension (302) est formé à partir d'un deuxième élément de tension.

4. Orthèse de membre, bandage de membre ou accessoire de sport selon la revendication 2 ou selon la revendication 3, **caractérisé en ce que** l'au moins un élément de tension est une corde, un fil, une bande plate ou un câble Bowden (320).

5. Orthèse de membre, bandage de membre ou accessoire de sport selon la revendication 4, **caractérisé en ce que** l'au moins un élément de tension comporte une corde ou un fil, dans lequel la corde ou le fil s'étend au moins par endroits dans une gaine (321), dans lequel la gaine est montée coulissante sur l'orthèse de membre.

6. Orthèse de membre, bandage de membre ou accessoire de sport selon l'une des revendications précédentes, dans lequel le premier élément d'accouplement (100) comprend une face avant (120) et une face arrière (130) et présente une forme en fer à cheval avec un bord intérieur concave (102) et dans lequel le deuxième élément d'accouplement (200) comprend une face avant (220) et une face arrière (230) et présente une forme en fer à cheval avec un bord intérieur concave (202), dans lequel le premier élément d'accouplement (100) comprend dans la zone du sommet (103) de la forme en fer à cheval sur la face avant (120) un premier élément d'encliquetage (104) et le deuxième élément d'accouplement (200) comprend dans la zone du sommet (203) de la forme en fer à cheval sur la face arrière (230) un deuxième élément d'encliquetage (204) et dans lequel le deuxième élément d'accouplement (200) en forme de fer à cheval comprend un premier élément formant pont (205) enjambant le bord intérieur concave (202) du deuxième élément d'accouplement (200), dans lequel, dans l'état relié des deux éléments d'accouplement (100, 200), le premier élément d'encliquetage (104) du premier élément d'accouplement (100) s'engage dans le premier élément formant pont (205) du deuxième élément d'accouplement (200) et le deuxième élément d'encliquetage (204) du deuxième élément d'accouplement (200) s'engage dans le bord intérieur concave (102) du premier élément d'accouplement (100).

7. Orthèse de membre, bandage de membre ou accessoire de sport selon l'une des revendications précédentes, **caractérisé en ce que** l'orthèse de membre comprend des guides de déviation (327) montés rotatifs dans lesquels l'au moins un élément de tension est dévié.

8. Orthèse de membre, bandage de membre ou accessoire de sport selon l'une des revendications précédentes, **caractérisé en ce que** l'orthèse ou le bandage de membre est une orthèse ou un bandage de genou (1000) ou l'accessoire de sport est un accessoire de sport pour le genou.

9. Orthèse de membre, bandage de membre ou accessoire de sport selon l'une des revendications précédentes, **caractérisé en ce que**, lorsque l'orthèse de membre est mise en place, le premier sous-système de tension (301) et/ou le deuxième sous-système de tension (302) du système de tension (300) sont guidés autour du membre, en particulier d'une jambe (1500), de telle sorte que le premier sous-système de tension (301) et/ou le deuxième sous-système de tension (302) du système de tension (300) entourent respectivement le membre.

10. Orthèse de membre, bandage de membre ou accessoire de sport selon l'une des revendications précédentes, **caractérisé en ce que** l'orthèse de membre comprend un élément de liaison auquel l'au moins un élément de tension, en particulier ledit élément de tension selon la revendication 2 ou le premier élément de tension et/ou le deuxième élément de tension selon la revendication 3 sont fixés.

11. Orthèse de membre, bandage de membre ou accessoire de sport selon la revendication 10, **caractérisé en ce qu'**un élément de tension selon la revendication 2 est fixé par les deux extrémités à l'élément de liaison.

12. Orthèse de membre, bandage de membre ou accessoire de sport selon la revendication 10 ou la revendication 11, **caractérisé en ce que** l'élément de liaison comprend un élément de réglage de mise en tension (340) pour la mise en tension de l'au moins un élément de tension.

13. Orthèse de membre, bandage de membre ou accessoire de sport selon l'une des revendications précédentes, **caractérisé en ce que** l'orthèse de membre, le bandage de membre ou l'accessoire de sport comprend au moins un rail articulé (410), de préférence deux rails articulés (410, 420).

14. Orthèse de membre, bandage de membre ou accessoire de sport selon la revendication 13, **caractérisé en ce que** l'au moins un rail articulé (410) comprend une première section de rail (411), une deuxième section de rail (412) et une troisième section de rail (413), dans lequel la première section de rail (411) et la deuxième section de rail (412) sont reliées respectivement par le biais d'une articulation (414, 415) à la troisième section de rail (413).

15. Orthèse de membre, bandage de membre ou accessoire de sport selon la revendication 14, **caractérisé en ce que** l'élément de liaison (340) selon l'une des revendications 10 à 12 est fixé à la troisième section de rail (413) de l'au moins un rail articulé (410).
